(19) Europäisches Patentamt / European Patent Office / Office européen des brevets

(11) **EP 2 645 934 B1**

(12) **EUROPÄISCHE PATENTSCHRIFT**

(45) Veröffentlichungstag und Bekanntmachung des Hinweises auf die Patenterteilung:
**13.03.2019 Patentblatt 2019/11**

(21) Anmeldenummer: **12713004.5**

(22) Anmeldetag: **23.03.2012**

(51) Int Cl.:
*A61B 6/03* $^{(2006.01)}$      *G01N 23/04* $^{(2018.01)}$
*G06T 11/00* $^{(2006.01)}$      *A61B 6/00* $^{(2006.01)}$
*G01N 23/046* $^{(2018.01)}$      *G06T 5/00* $^{(2006.01)}$
*G06T 5/50* $^{(2006.01)}$

(86) Internationale Anmeldenummer:
**PCT/EP2012/055202**

(87) Internationale Veröffentlichungsnummer:
**WO 2012/130754 (04.10.2012 Gazette 2012/40)**

(54) **VERFAHREN UND VORRICHTUNG ZUR KORREKTUR VON ARTEFAKTEN BEI EINER RÖNTGENBILDERZEUGUNG, INSBESONDERE COMPUTERTOMOGRAPHIE, ODER EINER RADIOGRAPHIE MITTELS ZEITLICHER MODULATION DER PRIMÄRSTRAHLUNG**

METHOD AND DEVICE FOR CORRECTING ARTEFACTS DURING X-RAY IMAGERY, ESPECIALLY COMPUTER TOMOGRAPHY, OR RADIOGRAPHY, BY MEANS OF TEMPORAL MODULATION OF THE PRIMARY RADIATION

PROCÉDÉ ET DISPOSITIF DE CORRECTION D'ARTÉFACTS EN IMAGERIE PAR RAYONS X, EN PARTICULIER EN TOMODENSITOMÉTRIE, OU EN RADIOGRAPHIE, PAR MODULATION DU RAYONNEMENT PRIMAIRE DANS LE TEMPS

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **01.04.2011 DE 102011006662**

(43) Veröffentlichungstag der Anmeldung:
**09.10.2013 Patentblatt 2013/41**

(73) Patentinhaber: **Siemens Aktiengesellschaft 80333 München (DE)**

(72) Erfinder:
• **SCHÖRNER, Karsten**
  **80802 München (DE)**
• **GOLDAMMER, Matthias**
  **80687 München (DE)**

(56) Entgegenhaltungen:
**DE-A1- 2 454 537**      **US-B2- 7 463 712**

• **KOWALSKI G: "Suppression of scattered radiation in radiography and improvement of resolution by spatially modulated intensity", APPLIED OPTICS USA, Bd. 15, Nr. 3, März 1976 (1976-03), Seiten 648-655, XP002678552, ISSN: 0003-6935**
• **GAO HEWEI ET AL: "Scatter correction method for x-ray CT using primary modulation: Phantom studies", MEDICAL PHYSICS, AIP, MELVILLE, NY, US, Bd. 37, Nr. 2, 28. Januar 2010 (2010-01-28), Seiten 934-946, XP012135617, ISSN: 0094-2405, DOI: 10.1118/1.3298014**

**Beschreibung**

Die vorliegende Erfindung betrifft ein Verfahren gemäß dem Oberbegriff des Hauptanspruchs und eine entsprechende Vorrichtung gemäß dem Oberbegriff des Nebenanspruchs.

In der Röntgen-Computertomographie (CT) tritt neben der zu detektierenden Primärstrahlung ebenso Streustrahlung auf. Wird die Detektion von Streustrahlung nicht verhindert, bzw. bleiben die aufgenommenen Projektionen unkorrigiert, führt dies zu Streustrahlartefakten in dem rekonstruierten CT-Volumen, das aus Voxeln besteht. Derartige Streustrahlartefakte können beispielsweise durch einen sogenannten Cupping-Effekt bewirkt sein, der zu ungleichmäßigen Voxelwerten in einem homogenen Objektmaterial führt, sodass sich bei Auftragung der Dichtewerte entlang einer Linie, also bei einer Erstellung eines Linienprofils, eine Wölbung anstelle einer Gerade ergibt. Zusätzlich können sich Streifenmuster und Kontrastverluste im Allgemeinen ergeben. Streustrahlung tritt ebenso im Bereich der digitalen Radiografie auf und bewirkt dort vor allem Kontrastverluste.

Zur Streustrahlungskorrektur bestehen unterschiedliche herkömmliche Lösungsansätze, die hauptsächlich in zwei Gruppen eingeteilt werden können:

1. Maßnahmen zur Reduktion der detektierten Streustrahlung, wie dies beispielsweise mittels Verwendung eines Anti-Streustrahlen-Gitters ausgeführt wird.

2. Sogenannte a posteriori-Korrekturen der Streustrahlung, indem in jeder CT-Projektion der Streuanteil entsprechend subtrahiert wird.

Für die 2. Gruppe ist eine möglichst genaue Kenntnis des detektierten Streuanteils erforderlich. Hierzu bestehen verschiedene Ansätze, diesen Streuanteil zu bestimmen, die ebenso in zwei Gruppen eingeteilt werden können:

1. Software-basierte Lösungen, wie dies beispielsweise Monte-Carlo-Simulationen, deterministische Berechnungen der Streuung erster Ordnung, Faltungsalgorithmen beruhend auf sogenannten Point-Spread-Functions sein können.

2. Experimentelle Methoden zur Bestimmung des Streuanteils anhand von Messungen.

Innerhalb dieser zweiten Gruppe der experimentellen Methoden sind unterschiedliche Messverfahren bekannt. Beispielsweise

a) Beam-Stopper basierte Verfahren und
b) eine dazu komplementäre Technik, die Aperturen und zwar sogenannte Beam-Holes verwendet und
c) ein erst vor kurzer Zeit vorgeschlagenes Verfahren, welches auf einer sogenannten Primärmodulation basiert.

Zu dem letzteren Verfahren ist folgender Stand der Technik bekannt.

Die US 7,463,712 B2 offenbart eine Streustrahlungskorrektur für eine Röntgenbilderzeugung, wobei eine richtungsabhängige Modulation der primären Röntgenstrahlung verwendet wird, die zu einer ortsabhängigen Modulation auf dem Detektor der Primärstrahlung führt. Eine Streustrahlung in einem eine Röntgenquelle und einen Röntgendetektor aufweisenden Röntgen-Bilderzeugungssystem wird unter Verwendung einer Amplitudenmodulation zur Übersetzung der Ortsfrequenz eines erfassten Röntgenstrahls zu einer höheren Frequenz und mittels Herausfilterung der niederfrequenten Streustrahlung korrigiert. Ein Maß für das niederfrequente Primärsignal ohne Streustrahlung wird danach mittels Demodulation erhalten.

Der entscheidende Vorteil eines Verfahrens mittels Primärmodulation im Vergleich zu vielen anderen herkömmlichen experimentellen Verfahren ist, dass hierbei die Streumessung und Streustrahlenabschätzung innerhalb des eigentlichen CT-Scans durchgeführt werden kann, d. h. die Streudaten werden simultan zu den eigentlichen CT-Projektionen gewonnen. Dies führt im Vergleich zu anderen herkömmlichen Verfahren, die einen zusätzlichen Messvorgang erfordern, zu einem kleineren Messaufwand und zugleich zu einer Zeitersparnis, welche insbesondere für die Röntgen-CT eine entscheidende Verbesserung darstellt. Zusätzlich wird auch Strahlungsdosis eingespart im Gegensatz zu Messverfahren, bei denen ein zusätzlicher Messvorgang erforderlich ist.

Das gemäß der US 7,463, 712 B2 offenbarte Verfahren beruht auf einer räumlichen Primärmodulation. Es ergibt sich aufgrund von hohen Bildfrequenzen, die insbesondere durch Kanten des aufzunehmenden Objekts bewirkte harte Übergänge im Bild verursacht sind, im Frequenzraum zu einer Überlappung der spektralen Kopie der Primärinformation mit der unmodulierten Frequenzinformation. Dies erschwert eine saubere Demodulation des Primärbildes. Dieses Problem wird mittels einer vorgeschlagenen sogenannten "boundary detection" teilweise gelöst. Des Weiteren ist man in der maximal auflösbaren (Bild-) Frequenz für die Rekonstruktion des Primärbildes, und somit ebenso für die des Streubildes, durch die feststehende, räumliche Modulatorfrequenz beschränkt. Die maximal rekonstruierbare Bildfrequenz für das Streubild liegt dabei bei der halben Modulatorfrequenz. Ein hier verwendeter unbewegter Primärmodulator führt häufig zu Ringartefakten in den rekonstruierten CT-Volumen.

Das herkömmliche Verfahren gemäß der US 7,463,712 B2 sieht vor, dass ein Primärmodulator zwischen das aufzunehmende Objekt und Röntgenröhre platziert wird. Der Primärmodulator prägt den Primärstrahlen per Amplitudenmodulation ein Muster auf, beispielsweise in Form eines Schachbrettes mit hellen und dunklen Feldern. Dazu kann beispielsweise eine Leiterplatine aus Kupfer verwendet werden, in die per Ätzprozesse ein Muster eingebracht wird, d. h. an den hellen Feldern wird das Kupfer entsprechend weggeätzt. Die unterschiedlich starken Abschwächungseigenschaften bzw. Abschwächungs-Koeffizienten von Kupfer bzw. von dem bloßen Leiterplatinen-Material sorgen für eine entsprechende Strahlabschwächung durch die dunklen Felder (Kupfer), während an den hellen Feldern kaum bzw. gar nicht abgeschwächt wird (Leiterplatinen-Material). Der Modulator befindet sich während des gesamten CT-Scans bzw. der gesamten CT-Abtastung stationär, das heißt, unbewegt, zwischen Objekt und Röntgenröhre, d. h., er verändert seine Position nicht. Das aufmodulierte, schachbrettartige Muster ist somit in jeder Projektion des CT-Scans wiederzufinden, d. h. sowohl in Freistrahlbereichen als auch in objektverdeckten Bereichen. Dabei ist die relative Modulationsstärke, was das Primärsignal angeht, an allen Stellen gleich groß. Aufgenommen wird jedoch vom Detektor nicht nur dieses modulierte Primärsignal, sondern zusätzlich wird dieses überlagert von einem unmodulierten, räumlich niederfrequenten Streusignal, das aufgrund von Röntgen-Streueffekten, inbesondere Compton-Streuprozessen, im Prüfobjekt und in der Laborumgebung zustande kommt. Der Detektor nimmt also ein Gesamtsignal auf, bestehend aus moduliertem Primärsignal und dem überlagerten unmodulierten Streusignal.

Im Folgenden ist es möglich, das modulierte Primärsignal im Fourier-Raum von dem unmodulierten Streusignal zu separieren. Dies erfolgt durch eine entsprechende Hochpass- bzw. Tiefpassfilterung der modulierten Projektion. Die tiefpassgefilterte Version der modulierten Projektion ergibt im Frequenzraum die Überlagerung der Frequenzanteile von unmoduliertem Primärbild und Streufunktion. Die hochpassgefilterte Version beinhaltet nur die spektrale Komponente des modulierten Primärsignals, kann also im Folgenden demoduliert und gewichtet werden, um im Frequenzraum eine Approximation des ausschließlichen Primärsignals zu erhalten. Dieses kann nach inverser Fourier-Transformation als approximiertes Primärbild von der tiefpassgefilterten Version, das Streu- und Primärsignale aufweist, subtrahiert werden, um eine Annäherung des Streubildes zu erhalten. Es wird darauf hingewiesen, dass bei dem hiermit beschriebenem Verfahren auf jede modulierte Projektion eine sogenannte Kantenerkennung und Kantenglättung, die ebenso als Boundary Detection bezeichnet wird, angewendet wird. Eine derartige Glättung berücksichtigt, dass alleine durch das Objekt und insbesondere durch die Objektkanten bereits hochfrequente Bildanteile vorhanden sind. Dies ist unabhängig von jeder Modulation. Diese hochfrequenten, unmodulierten Anteile überlappen im Fourier-Raum mit den spektralen Kopien des modulierten Primärsignals. Eine Vermischung von moduliertem und unmoduliertem Signal hat, falls dieses nicht korrigiert wird, eine nicht korrekte Demodulation der Primärsignale zur Folge. Das heißt, es kommt besonders in den Objektkantenbereichen und ebenso im Objektinneren, was dann in diesem Zusammenhang als "Spilling" bezeichnet wird, zu Artefakten und einer falschen Rekonstruktion des Primärbildes. Diese Effekte wirken sich ebenso in dem später erzeugten Streubild aus. Um derartige hochfrequente Bildanteile, die insbesondere durch Objektkanten verursacht werden, zu dämpfen, bzw. zu unterdrücken, wird die sogenannte Boundary Detection angewendet, die Objektkanten findet und diese entsprechend mit einem Gaußschen Filter glättet. Hierbei werden zwar die starken, durch zu hochfrequente, unmodulierte Bildanteile verursachten Artefakte verringert, allerdings ergibt sich eine Ungenauigkeit im Kantenbereich an derartigen Stellen, da die Gauß-Filterung später nicht mehr rückgängig gemacht wird. Das so gewonnene Streubild wird nun von den CT-Projektionen entsprechend abgezogen. Da jetzt noch das Modulationsmuster in den Projektionen vorhanden ist, werden die CT-Projektionen auf die Strahlungsintensität nach dem Modulator normiert. Auf diese Weise kann dann das Modulatormuster im Projektionsbild entfernt werden. Dies erfolgt mittels Division durch eine Aufnahme des Modulators ohne weitere Objekte im Strahlengang. Eine derartige Aufnahme kann ebenso als Referenzmessung bezeichnet werden. Es wird darauf hingewiesen, dass Strahlaufhärtungseffekte, die ebenso Beam Hardening Effekte genannt werden, durch den Modulator entstehen, und zwar insbesondere durch die dunklen Kupferfelder. Verbleiben derartige Effekte unkorrigiert, ergibt sich erstens eine fehlerhafte Streuabschätzung und zweitens eine nicht vollständige Beseitigung des Schachbrettmusters im letztgenannten Divisionsschritt. Wegen des stationären Modulators kann dies Ringartefakte in den CT-Querschnittsbildern zur Folge haben. Dass Strahlaufhärtungseffekte existieren und zu den genannten Fehlern führen, ist beispielsweise aus der [1] bekannt.

[1] offenbart, dass bei Verwendung von polyenergetischen Röntgenquellen infolge von auf fehlerhaften Gradienten von linearen Abschwächungskoeffizienten in Computertomographie-Querschnittsbildern beruhender Strahlaufhärtung genaue Dichtemessungen erschwert sind. Es wird ein Korrekturverfahren beschrieben, bei dem mittels Linearisierung polyenergetische in monoenergetische Computertomographiedaten umgewandelt werden. Es werden Computertomographiedaten als eine Funktion der Objektdicke aus gemessenen Datenpunkten abgeleitet und als Polynom dargestellt. Mittels Simulation werden auf der Grundlage der Objektmaterialdichte, der Objektmaterialzusammensetzung, des Röntgenenergiespektrums, der Detektorantwort und des Informationstransfers vom Detektor zu digitalisierten Daten die polyenergetischen Computertomographiedaten genau simuliert. Die Bogenlinie der die polyenergetischen Computertomographiedaten darstellenden Funktion kann mittels eines Polynoms achter oder höherer Ordnung oder mittels Cubic-Spline-Interpolation genau bestimmt werden.

Die DE 24 54 537 offenbart ein Verfahren zur Reduzierung des Einflusses von Streustrahlung und eine Anordnung zur Durchführung des Verfahrens, wobei bei einer Aufnahmeanordnung mit einer Quelle inkohärenter Strahlung, vorzugsweise einem Röntgenstrahler, ein periodisches Gitter zwischen der Quelle und ein Aufnahmegerät zur Aufnahme des Schattenbildes eines zu untersuchenden Objektes, vorzugsweise zwischen die Quelle und das Objekt, geschoben wird, so dass die niedrigen Ortsfrequenzen des mit dem Gitter aufgenommenen Bildes des Objekts unterdrückt werden und dass die durch das Gitter modulierte Bildinformation anschließend demoduliert wird.

Kowalski, G.: "Suppression of scattered radiation in radiography and improvement of resolution by spatially modulated intensity", in Applied Optics USA, Band. 15, Nr. 3, März 1976 (1976-03), Seiten 648-655, XP002678552, ISSN: 0003-6935 offenbart bei einer Radiographie einer punktförmigen Röntgenquelle Wege zur Bereitstellung einer Hochauflösung mittels elektronischer Demodulation.

Es ist Aufgabe der vorliegenden Erfindung ein Verfahren und eine Vorrichtung zur Röntgenbilderzeugung, insbesondere Computertomographie, oder digitalen Radiographie derart bereit zu stellen, dass infolge einer Streustrahlung erzeugte Artefakte in einem rekonstruierten Computertomographievolumen im Vergleich zum Stand der Technik einfacher und wirksamer korrigiert werden. Derartige Artefakte können beispielsweise Cupping-Effekte, Streifenmuster und/oder Kontrastverluste sein. Des Weiteren soll zudem ein Artefakt einer infolge einer Primärmodulation bewirkten Strahlaufhärtung einfach und wirksam korrigiert werden. Beispielsweise soll eine Kontrastverbesserung im Vergleich zu herkömmlichen Verfahren bewirkt werden.

Die Aufgabe wird durch ein Verfahren gemäß dem Hauptanspruch und eine Vorrichtung gemäß dem Nebenanspruch gelöst.

Gemäß einem ersten Aspekt wird ein Verfahren zur Korrektur von Artefakten in einer Röntgenprojektion eines Objektes bereitgestellt, wobei eine Röntgenstrahlung einer primären Röntgenquelle mit einem sich wiederholenden Muster von Bereichen mit unterschiedlicher Röntgenstrahlungssignalstärke beaufschlagt, dadurch amplitudenmoduliert wird, danach durch das abzubildende Objekt zu einem Detektor verläuft, dort als Gesamtsignale erfasst und daraus ein Streubild berechnet wird, das von einer ursprünglichen amplitudenmodulierten Projektion getrennt wird. Dabei ist vorgesehen, dass die Röntgenstrahlung der primären Röntgenquelle Primärsignale erzeugt und diese zeitlich veränderlich amplitudenmoduliert werden, so dass eine Anzahl $j = 1 ... N_{Mod}$ von einem jeweiligen Zeitpunkt zugeordneten Modulationsprojektionen erzeugt wird. Das Verfahren zeichnet sich dadurch aus, dass zur Berechnung eines einer Projektion zugeordneten Streubildes für jeden Detektorpixel ein Streusignal des Detektorpixels mittels Subtraktion eines nach der zeitlichen Modulation demodulierten Primärsignals des Detektorpixels von dem Gesamtsignal des Detektorpixels erhalten wird und alle auf diese Weise ermittelten Streuwerte für alle Detektorpixel zusammengesetzt das Streubild ergeben.

Gemäß einem zweiten Aspekt wird ein Computertomograph zur Korrektur von Artefakten in einer Röntgenprojektion eines Objekts bereitgestellt, wobei eine Röntgenstrahlung einer primären Röntgenquelle durch ein Modulatorfeld mit einem sich wiederholenden Muster von Bereichen mit unterschiedlicher Röntgenstrahlungsabschwächung verläuft, dadurch amplitudenmoduliert wird, danach durch das abzubildende Objekt zu einem Detektor verläuft, dort erfasst und daraus ein Streubild berechnet wird, das von einer ursprünglichen amplitudenmodulierten Projektion getrennt wird, wobei das Modulatorfeld ein sich wiederholendes Muster aufweist, dessen erste Hälfte deckungsgleich mit einer zweiten Hälfte ist, zueinander deckungsgleiche Bereiche der beiden Hälften zueinander entgegen gesetzte Röntgenstrahlungs-Abschwächungskoeffizienten aufweisen, sich das Muster entlang mindestens einer Wiederholungslinie wiederholt und eine Länge des Musters entlang der Wiederholungslinie einer Periodenlänge entspricht. Die Vorrichtung zeichnet sich dadurch aus, dass eine Verschiebeeinrichtung zu einer relativen Bewegung der Röntgenquelle, des Objekts und des Detektors einerseits und des Modulatorfeldes andererseits von einer ersten relativen Position in eine zweite relative Position entlang der Wiederholungslinie um jeweils ein ungeradzahliges Vielfaches einer halben Periodenlänge das Modulatorfeld derart verschiebt, dass Modulatorfeldbereiche mit kleinem und dazu relativ großem Röntgenstrahlungs-Abschwächungskoeffizienten gegenseitig ausgewechselt werden.

Ein als ein Primärmodulator wirkendes Modulatorfeld erstreckt sich entlang einer Ebene und weist dem Modulatormaterial entsprechende Dicken auf.

Entgegengesetzte Röntgenstrahlungsabschwächungskoeffizienten bedeutet beispielsweise einmal einen großen und einen zu diesem relativ kleinen Röntgenstrahlungsabschwächungskoeffizienten. Ein Wertebereich von normierten Röntgenstrahlungsabschwächungskoeffizienten ist dadurch bestimmt, dass diese Werte insbesondere größer gleich null und kleiner gleich eins aufweisen können.

Es ergeben sich Modulations-Projektionsbildfelder, die durch relativ große Röntgenstrahlungssignalstärken erzeugt wurden, die als helle Felder bezeichnet werden können. Modulations-Projektionsbildfelder, die durch im Vergleich zu den großen Röntgenstrahlungssignalstärken kleinere Signale erzeugt wurden, können als dunkle Felder bezeichnet werden. Große Strahlungsintensität erzeugt ein großes Signal, hohen Grauwert. Kleine Strahlungsintensität erzeugt ein kleineres Signal, also kleineren Grauwert.

Eine Wiederholungslinie des Musters des Modulatorfeldes ist eine Linie, entlang der sich das Muster wiederholt. Eine Länge einer Wiederholungslinie in einem Muster entspricht einer Periodenlänge. Eine Wiederholungslinie kann eine Gerade sein.

Vorteilhaft können gemäß der erfindungsgemäßen Lösung aufgrund dunkler Gitterfelder in einem statischen Primärmodulator erzeugte Ringartefakte wirksam verringert werden.

Des Weiteren hat ein bewegter Primärmodulator im Vergleich zu einem stationären Primärmodulator den großen Vorteil, dass etwaige nicht vollständig kompensierte Restmuster des Primärmodulators bei einem Rückprojektionsprozess der Rekonstruktion gleichmäßig auf das gesamte Volumen bzw. gleichmäßig über einen CT-Querschnitt verteilt werden. Auf diese Weise können typische Ringartefakte, wie sie bei einem stationären Modulator auftreten, vermieden werden. Auf diese Weise ist ebenso eine nachgeordnete Verwendung von Algorithmen zur Unterdrückung von Ringartefakten, wie es gemäß der US 7,463,712 B2 beschrieben ist, lediglich fakultativ.

Gemäß der vorliegenden Erfindung ergeben sich folgende Vorteile: Das Problem wird dadurch gelöst, dass eine Überlappung von modulierten und unmodulierten Frequenzbereichen bei der zeitlichen Modulation vermieden wird, da das Nutzsignal, das einer Abschwächung des Primärsignals durch das Objekt entspricht, zeitlich konstant und somit auf eine Frequenz von =0 beschränkt ist. Des Weiteren kann ein erfindungsgemäßes Verfahren für jeden Detektorpixel einzeln ausgeführt werden. Auf diese Weise kann die maximal auflösbare Frequenz bis zur durch den Detektor vorgegebenen Maximalfrequenz angehoben werden. Das heißt, dass die maximale Auflösung für die Generierung des Streubildes zur Verfügung steht. Des Weiteren können zur Vermeidung von Ringartefakten etwaige Restfehler, beispielsweise in Folge einer unzureichenden Strahlaufhärtungskorrektur oder bei der Division der Modulatorprojektion durch die Referenzprojektion ohne Objekt, beim Rückprojektionsprozess der Rekonstruktion gleichmäßiger auf das gesamte Volumen bzw. gleichmäßiger über einen CT-Querschnitt verteilt werden, so dass typische Ringartefakte, wie sie beim unbewegten Modulator entstehen, kaum bzw. gar nicht auftreten. Dadurch ist ebenso ein nachfolgender Einsatz von Algorithmen zur Unterdrückung von Ringartefakten lediglich fakultativ.

Gemäß der vorliegenden Erfindung kann besonders vorteilhaft jeweils für einen Pixel ein zeitlich moduliertes Signal bereitgestellt und einfach weiterverarbeitet werden.

Weitere vorteilhafte Ausgestaltungen werden in Verbindung mit den Unteransprüchen beansprucht.

Gemäß einer vorteilhaften Ausgestaltung kann die primäre Röntgenquelle einzeln ansteuerbare Matrixbereiche aufweisen, die zu einem vorangehenden Zeitpunkt entsprechend dem Muster eine kleine oder eine dazu relativ große und zu einem nachfolgenden Zeitpunkt die jeweils andere Röntgenstrahlungssignalstärke aussenden und zu jedem der Zeitpunkte jeweils eine ursprüngliche amplitudenmodulierte Modulationsprojektion des Objekts erzeugt und jeweils ein dieser Modulationsprojektion zugeordnetes Streubild ermittelt wird.

Gemäß einer weiteren vorteilhaften Ausgestaltung kann im Strahlenverlauf von der Röntgenquelle zum Objekt ein dem Muster entsprechende Bereiche mit einer kleinen oder einer dazu relativ großen Röntgenstrahlungsabschwächung aufweisendes Modulatorfeld angeordnet sein, und Röntgenquelle, Objekt und Detektor einerseits und das Modulatorfeld andererseits von einer vorangehenden Position in eine nachfolgende Position relativ zueinander bewegt werden und dadurch Modulatorfeldbereiche mit kleinem und dazu relativ großem Röntgenstrahlungs-abschwächungskoeffizienten im Röntgenstrahlungsverlauf gegenseitig ausgewechselt werden, in jeder der beiden Positionen jeweils eine ursprüngliche amplitudenmodulierte Modulationsprojektion des Objekts erzeugt und jeweils ein diesen Modulationsprojektionen zugeordnetes Streubild ermittelt wird. Aus einem Satz an Modulationsprojektionen j=1 ... N_ModProj ergibt sich ein Streubild. Dieses kann im Folgenden von den Modulationsprojektionen einzeln abgezogen werden, bevor diese dann BHC- (Strahlaufhärtungskorrektur-) korrigiert und durch die Modulationsfunktion geteilt werden.

Es sind außer den Maximalstufen, d.h. ganz dunkles oder ganz helles Feld auch Zwischenstufen denkbar, also eine wirkliche Modulation entsprechend einem sinusförmigen Verlauf. Dies könnte z.B. durch einen graduell geprägten Primärmodulator, also nicht nur Schachbrettmuster, sondern feinere Abstufungen zwischen den Feldern, erreicht werden.

Gemäß der Erfindung wird ein Streusignal eines Detektorpixels oder ein Streubild eines Detektors mittels Subtraktion eines nach der zeitlichen Modulation demodulierten Primärbildes von dem Gesamtbild erhalten.

Gemäß einer weiteren vorteilhaften Ausgestaltung wird die Demodulation mittels Multiplikation des aufgenommenen, teils modulierten, Gesamtsignals mit einer Modulationsfunktion ausgeführt wird. Dies entspricht einer Multiplikation von zeitlichen Signalen. Gemäß der Erfindung werden ausgehend von zeitlichen Signalen nach entsprechender Fouriertransformation Frequenzen erzeugt, die zeitlich bezogen sind.

Gemäß einer weiteren vorteilhaften Ausgestaltung kann eine zur Bereitstellung einer streukorrigierten Modulationsprojektion erfolgende Subtraktion des der Modulationsprojektion zugeordneten Streubildes von einer dazugehörigen ursprünglichen, nicht unterabgetasteten Modulationsprojektion, ausgeführt werden.

Gemäß einer weiteren vorteilhaften Ausgestaltung kann eine zur Bereitstellung einer zusätzlich modulationskorrigierten Modulationsprojektion erfolgende Division der streukorrigierten Modulationsprojektion durch eine ohne das Objekt ermittelte zeitliche Modulationsfunktion ausgeführt werden.

Gemäß einer weiteren vorteilhaften Ausgestaltung kann ein Erzeugen jeweils einer streukorrigierten oder streu- und modulatorfeldkorrigierten Modulationsprojektion jeweils für eine Anzahl j = 1 ...$N_{Mod}$ Modulationen mit darauf folgender Mittelung zur Vergrößerung eines Signal-Rausch-Verhältnisses ausgeführt werden.

Gemäß einer weiteren vorteilhaften Ausgestaltung können die vorangehende und nachfolgende Modulation jeweils eine Modulationsprojektion j=1 und j=2 erzeugen und danach Röntgenquelle und Detektor einerseits und das Objekt andererseits nach jeder zweiten Modulationsprojektion um eine Drehachse in eine Drehrichtung um einen Drehwinkelschritt von einer Relativposition i in eine Relativposition i+1 relativ zueinander gedreht werden.

Gemäß der Erfindung ist ein Konstruieren eines modulatorfreien Gesamtbildes nicht notwendig. Dies ist dadurch begründet, dass eine Modulation und Demodulation nicht zwischen Orts-/Bildraum und dazugehörigem Frequenzraum ausgeführt wird, sondern eine zeitliche Modulation derart ausgeführt wird, dass Räume Zeit und zugehöriger Frequenzraum sind.

Gemäß einer weiteren vorteilhaften Ausgestaltung kann zur Berechnung eines einer Modulationsprojektion zugeordneten Streubildes ein Unterabtastungsschritt ausgeführt werden. Gemäß einem erfindungsgemäßen Verfahren kann das Streubild pixelgenau ermittelt werden. Zur Rauschunterdrückung kann davor optional ein Unterabtasten beziehungsweise Downsampling der Modulationsprojektionen erfolgen. Aufgrund der erfindungsgemäßen zeitlichen Modulation und Demodulation werden gegebene Ortfrequenzen entfernt, das heißt ebenso die von einem Primärmodulator vorgegebene räumliche Modulationsfrequenz. _Aufgrund dessen kann eine erfindungsgemäße pixelgenaue Berechnung eines Streuwertes ausgeführt werden.

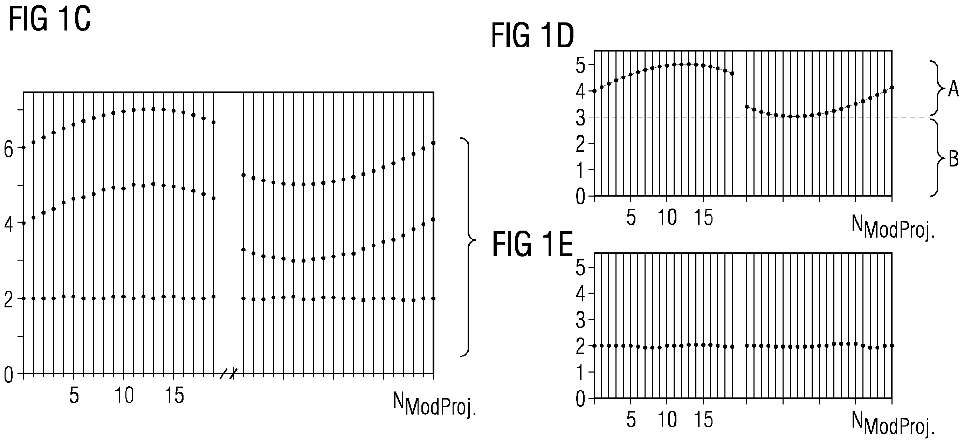

Gemäß einer weiteren vorteilhaften Ausgestaltung kann zur Berechnung eines einer Modulationsprojektion zugeordneten abgeschätzten Primärbildes, bestehend aus einzelnen Primärsignalen, pro Pixel das, insbesondere teilweise modulierte, Gesamtsignal über alle Modulationsprojektionen j=1...N_ModProj hinweg als zeitliches Signal gewertet und dieses hochpassgefiltert werden, wodurch unmodulierte Anteile wegfallen. Unmoduliert sind ein Großteil, beispielsweise ca. 80%, des Primärsignals, wobei auf Figur 1d, Signal B verwiesen wird, sowie das komplette Streusignal, wobei diese Annahme, Figur 1e darstellt; danach mittels Multiplikation mit der jeweiligen dem Pixel eigenen Modulationsfunktion, die ohne Objekt aufgenommen werden kann, demoduliert und danach Tiefpass gefiltert werden.

Gemäß einer weiteren vorteilhaften Ausgestaltung kann zur Berechnung eines einer Modulationsprojektion zugeordneten Streubildes das der Modulationsprojektion zugeordnete demodulierte Primärbild von dem Gesamtbild subtrahiert werden. Dabei kann ein gemitteltes Gesamtbild verwendet werden, das sich durch Addition der Modulationsprojektionen und entsprechende Gewichtung ergibt. Ein demoduliertes Primärsignal eines Pixels kann von einem Gesamtsignal des Pixels subtrahiert werden. Ein Gesamtsignal kann mittels Mittelung von allen Gesamtsignalen an diesem Pixel für alle Modulationsprojektionen erhalten werden. Alle Streusignale für die Pixel werden zu einem Streubild zusammengesetzt, das von den Modulationsprojektionen subtrahiert und weiterverarbeitet werden kann.

Gemäß einer weiteren vorteilhaften Ausgestaltung kann zur Strahlaufhärtungskorrektur jeweils für ein Modulationsprojektionsbildfeld, das durch eine relativ kleine Röntgenstrahlungssignalstärke erzeugt wurde, von einem gemessenen unkorrigierten Intensitätswert ein angenäherter Streuwert subtrahiert werden, diese Differenz einem ersten Intensitätswert entspricht, der mittels einer dazugehörigen Intensitätsabschwächungskurve einer dazugehörigen durchstrahlten Objektlänge zugeordnet werden kann, diese mittels einer Intensitätsabschwächungskurve für ein Modulationsprojektionsbildfeld, das durch eine relativ große Röntgenstrahlungssignalstärke erzeugt wurde, einem zweiten Intensitätswert zugeordnet werden kann, zu dem der angenäherte Streuwert wieder addiert werden kann.

Gemäß einer weiteren vorteilhaften Ausgestaltung kann der angenäherte Streuwert aus einem berechneten Streubild einer vorangegangenen ursprünglichen Modulationsprojektion ermittelt werden.

Gemäß einer weiteren vorteilhaften Ausgestaltung kann die Strahlaufhärtungskorrektur während eines Unterabtastschrittes nach Anspruch 11 ausgeführt werden.

Gemäß einer weiteren vorteilhaften Ausgestaltung kann die Strahlaufhärtungskorrektur vor der Division der streukorrigierten Modulationsprojektion durch die ohne das Objekt ermittelte zeitliche Modulationsfunktion nach Anspruch 7 ausgeführt werden.

Gemäß einer weiteren vorteilhaften Ausgestaltung können die Verschiebeeinrichtung und eine Dreheinrichtung zum Drehen des Objekts um eine Drehachse in eine Drehrichtung um jeweilige Drehwinkelschritte miteinander synchronisiert sein.

Gemäß einer weiteren vorteilhaften Ausgestaltung kann das Muster ein Linien-, Viereck-, Wellen- oder Radialmuster sein.

Gemäß einer weiteren vorteilhaften Ausgestaltung können sich Röntgenstrahlungsabschwächungskoeffizienten von Übergängen der Modulatorfeldbereiche mit kleinem zu den Modulatorfeldbereichen mit relativ großem Röntgenstrahlungsabschwächungskoeffizienten kontinuierlich und stetig verändern.

Gemäß einer weiteren vorteilhaften Ausgestaltung können Modulationsstärken der Modulatorfeldbereiche mit relativ großem Röntgenstrahlungsabschwächungskoeffizienten eine Gesamtsignal-Verringerung in einem Bereich von 10% bis 30% bewirken.

Gemäß einer weiteren vorteilhaften Ausgestaltung kann das Modulatorfeld Bereiche mit kleinem und dazu relativ großem sowie mit zusätzlichen Röntgenstrahlungsabschwächungskoeffizienten aufweisen.

Die vorliegende Erfindung wird anhand von Ausführungsbeispielen in Verbindung mit den Figuren näher beschrieben. Es zeigen:

Figur 1a        eine Darstellung von i CT-Projektionen auf ein relativ bewegtes Objekt;

Figur 1b        eine Darstellung von j Modulationsprojektionen, die jeweils einer CT-Projektion zugeordnet sind;

Figur 1c        eine Darstellung einer diskret abgetasteten Aufnahme eines Gesamtsignals;

Figur 1d        eine Darstellung eines modulierten und eines unmodulierten Anteils eines Primärsignals aus Figur 1c;

Figur 1e        eine Darstellung eines Streusignals eines Primär-signals aus Figur 1c;

Figur 2        ein Ausführungsbeispiel einer Berechnung eines Streuwertes für ein Pixel;

Figur 3a bis 5b        jeweils ein Ausführungsbeispiel für ein erfindungsgemäßes Modulatorfeld;

Figur 6        ein Ausführungsbeispiel von Modulatorfeldbewegungen und dazu relativen Objektbewegungen;

Figur 7        ein Ausführungsbeispiel einer Korrektur von Strahlaufhärtungseffekten;

Figur 8        ein Ausführungsbeispiel einer erfindungsgemäßen Vorrichtung.

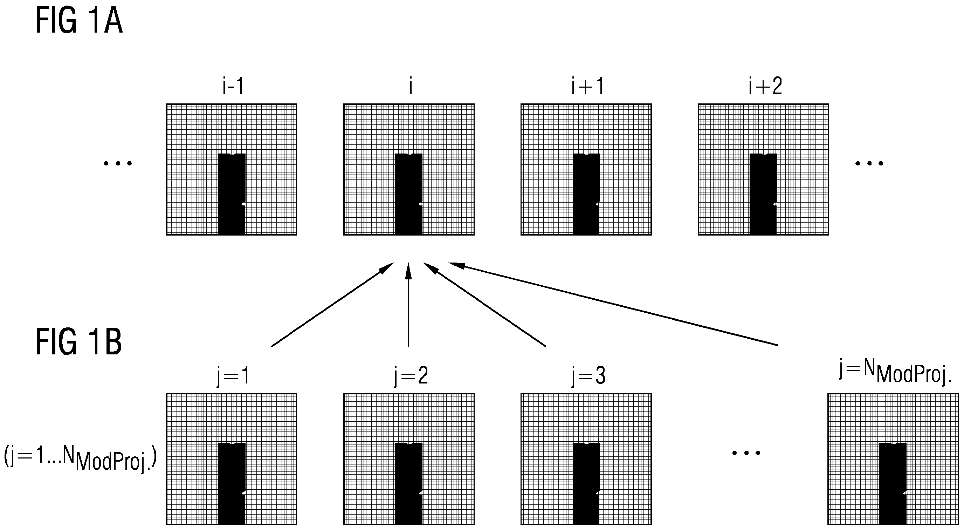

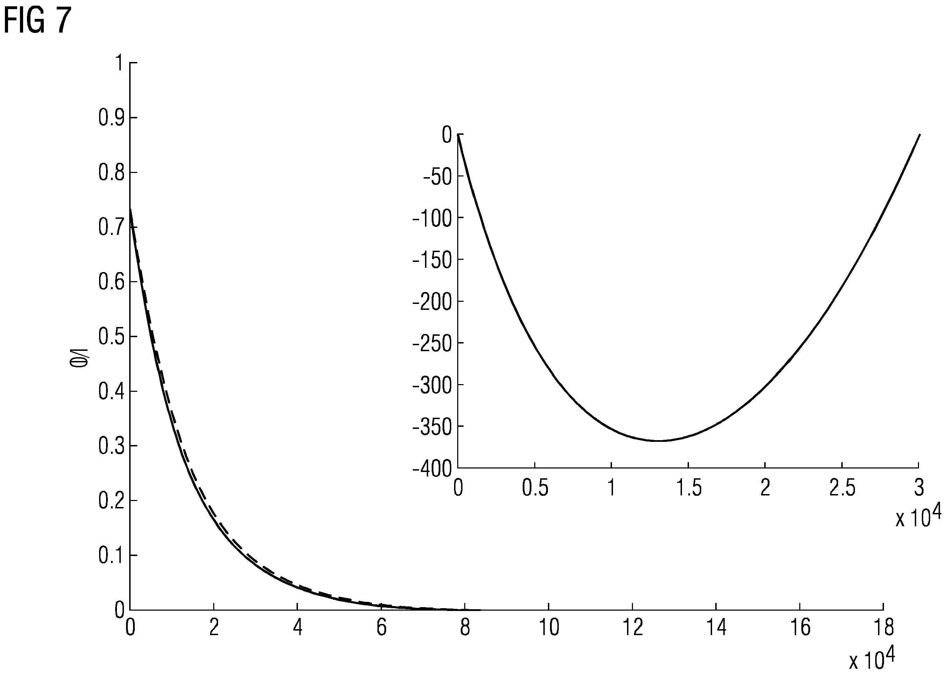

Figur 1a zeigt eine Darstellung von CT-Projektionen auf ein relativ bewegtes Objekt. Ein erfindungsgemäßes Verfahren zur Streustrahlkorrektur beruht auf einer zeitlichen Modulation eines Primärsignals. Es wird von einem CT-Scan bzw. einer CT-Abtastung ausgegangen, bei der i=1... $N_{Proj}$ CT-Projektionen aufgenommen werden. Das heißt beispielsweise, dass das zu tomografierende Objekt $N_{Proj}$ - mal gedreht wird, und zwar insbesondere bei der industriellen CT, oder aber Röhre und Detektor werden $N_{Proj}$ - mal bewegt und zwar insbesondere bei der medizinischen CT oder bei der industriellen CT für große Objekte, um das zu tomografierende Objekt aus $N_{Proj}$ - verschiedenen Projektionsrichtungen zu durchstrahlen. Die Projektionsbilder werden dabei von einem Flächendetektor/Zeilendetektor zweidimensional oder eindimensional aufgenommen. Ein derartiger Detektor besteht aus Pixeln (x, y) mit x=1... $x_{Pix}$ und y=1...$y_{Pix}$. Für eine weitere allgemeine Beschreibung wird lediglich ein Pixel aus dieser Menge, das im Folgenden als (x', y') bezeichnet wird, betrachtet. Bis auf weiteres werden Strahlaufhärtungseffekte vernachlässigt, d. h. es wird von einer monochromatischen Strahlung ausgegangen. Eine Erweiterung auf polychromatische Strahlung wird in Verbindung mit Figur 7 vorgenommen. Für eine Streustrahlenmessung wird jede CT-Projektion i in $N_{ModProj}$ Einzelprojektionen unterteilt, die ebenso als Modulationsprojektionen bezeichnet werden können. Diese Einzelprojektionen werden mit j durchnummeriert, wobei j=1... $N_{ModProj}$ ist, und innerhalb derer eine zeitliche Modulation des Primärsignals für jeden Pixel (x, y) erfolgt.

Figur 1b zeigt eine Darstellung von j Modulationsprojektionen, die jeweils einer CT-Projektion zugeordnet sind. Bei einem derartigen Satz von Modulationsprojektionen ist das Objekt bei einer industriellen CT bzw. sind Röhre und Detektor bei einer medizinischen CT stationär, d. h. unbewegt. Lediglich das Primärsignal wird zeitlich amplitudenmoduliert, und zwar derart, dass der detektierte Streuanteil, der hauptsächlich von Compton-Röntgenstreu-Prozessen verursacht wird, und zwar insbesondere in dem zu tomografierenden Objekt und im kleineren Ausmaß ebenso aufgrund der Laborumgebung, und deren räumliche Verteilung sich möglichst wenig ändert. Letztere Forderung stellt gewisse Bedingungen an die technische Realisierung der Primärmodulation, die gemäß der vorliegenden Erfindung erfüllt werden können.

Figur 1c zeigt eine Darstellung einer diskret abgetasteten Aufnahme eines Gesamtsignals. Beispielsweise können für eine CT-Projektion i die Vielzahl von Modulationsprojektionen j=1... $N_{ModProj}$ nun für jeden einzelnen Pixel (x', y') die diskrete bzw. gesamplete bzw. unterabgetastete Aufnahme des Gesamtsignals abbilden. Die Graphen der Figuren 1c bis 1e zeigen einen Signalverlauf in einer beliebigen Einheit gegenüber der Zeit in Einheiten der j Modulationsprojektionen. Figur 1c stellt die in den jeweiligen Modulationsprojektionen erfolgende zeitliche Modulation des Primärsignals betrachtet für lediglich einen Pixel (x', y') dar. Die diskrete Aufnahme des Gesamtsignals besteht aus dem modulierten Primär- und dem überlagernden, unmodulierten Streusignal wie es die Figuren 1c bis 1e darstellen. Figur 1c zeigt eine Darstellung einer diskret abgetasteten Aufnahme des Gesamtsignals für das Pixel, wobei das aus Primär- und Streusignal bestehende Gesamtsignal, als die obere Kurve dargestellt ist. Die mittlere Kurve stellt das Primärsignal und die untere Kurve stellt das Streusignal dar. In Figur 1d ist ein modulierter und ein unmodulierter Anteil des Primärsignals aus Figur 1c dargestellt. Mit A ist der modulierte Primäranteil gekennzeichnet, mit B ist der unmodulierte Primäranteil gekennzeichnet. Figur 1e stellt das Streusignal, also eines Streuanteils eines Gesamtsignals aus Figur 1c getrennt dar. Ein Gesamtsignal für ein Pixel (x', y') entspricht dem gemessenen Grauwert dieses Pixels.

Figur 1e zeigt eine Darstellung eines Streusignals, also eines Streuanteils eines Gesamtsignals aus Figur 1c in einem separaten Graphen.

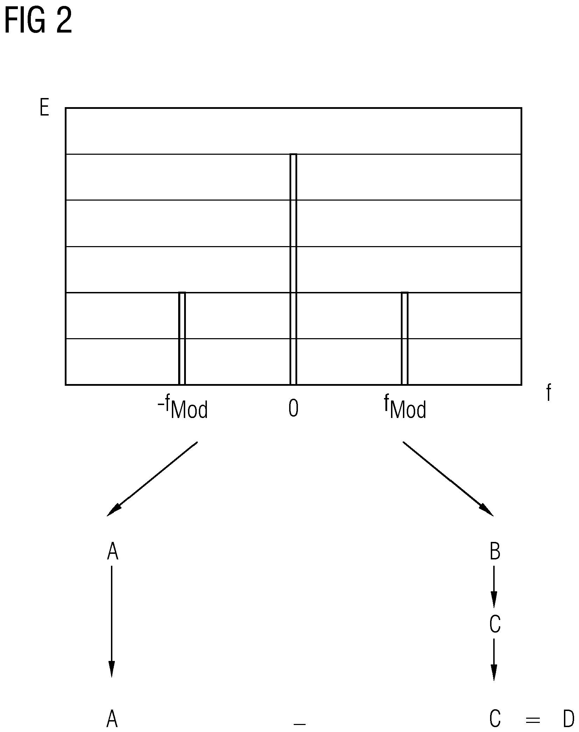

Figur 2 stellt ein Ausführungsbeispiel einer Berechnung eines Streuwertes für einen Pixel dar. Figur 2 stellt in einem Graph mit einer Frequenz-Abszisse und einer Energie-Ordinate ein Spektrum des fouriertransformierten Gesamtsignals eines Pixels (x', y') dar. Dabei entspricht ein Energieanteil A bei einer Frequenz =0 einem Streuanteil und einem unmodulierten Primäranteil. Ein Energieanteil B bei einer Modulationsfrequenz entspricht dabei einem modulierten Primäranteil. Dieser Energieanteil B wird entsprechend einer Modulationsstärke demoduliert und gewichtet, so dass ein demoduliertes, gewichtetes Primärsignal C erhalten wird. Durch die entsprechende Demodulation des zeitlich modulierten Primärsignals und durch anschließende Subtraktion dieses demodulierten Primärsignals C von dem gemessenen Gesamtsignal A kann der Streuwert B für das entsprechende Pixel (x', y') berechnet werden. Alle auf diese Weise ermittelten Streuwerte für alle Pixel ergeben zusammengesetzt das sogenannte Streubild. Das Streubild kann, nach einer entsprechenden Weiterverarbeitung, wie es beispielsweise eine Glättung ist, von den j Modulationsprojektionen abgezogen werden. Die Modulationsprojektionen sind damit streukorrigiert. Lediglich die zeitliche Modulation des Primärsignals ist für jeden einzelnen Pixel in den Modulationsprojektionen dann noch vorhanden. Durch eine vorangegangene Referenzmessung ohne Objekt, bei der ausschließlich die zeitliche Modulationsfunktion aufgenommen wird, kann in den Modulationsprojektionen diese nun herausdividiert werden. Zur Verbesserung eines Signal-Rausch-Verhältnisses (SNR) werden die streu- und modulationskorrigierten Projektionen dann gemittelt, da sie alle das zu tomografierende Objekt an der gleichen Position abbilden. Auf diese Weise kann eine vollständig korrigierte CT-Projektion i erhalten werden.

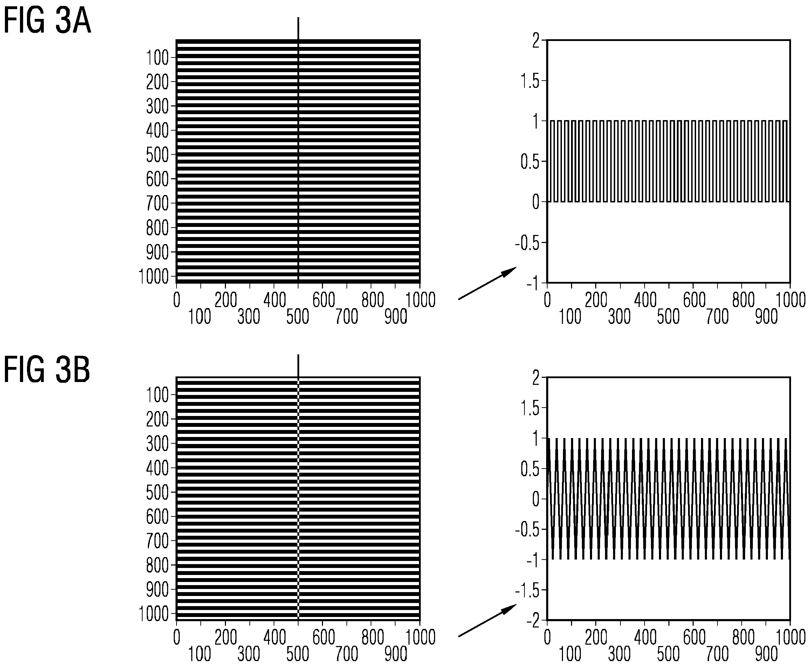

Figuren 3a bis 5b zeigen Ausführungsbeispiele eines erfindungsgemäßen Modulatorfeldes 3, insbesondere aus der Richtung einer primären Röntgenquelle betrachtet. Eine erfindungsgemäße zeitliche Modulation des Primärsignals kann beispielsweise mittels eines relativ zum aufzunehmenden Objekt bewegten Primärmodulators ausgeführt werden. Ein derartiger Primärmodulator ist ein Modulatorfeld, das ein entsprechendes Strahlabschwächungsmuster aufweist. Dies kann beispielsweise ein schachbrettartiges Muster sein. Durch einen entsprechenden Versatz, beispielsweise in horizontaler oder vertikaler Richtung, dieses Modulatorfeldes kann für jeden Pixel auf dem Detektor das Abschwächungsmuster von hellen Feldern, d. h. keine zusätzliche Abschwächung, zu dunklen Feldern, d. h. eine gewisse Strahlabschwächung, variiert werden. Dies stellt aber zunächst lediglich eine mögliche Ausführungsform dar, weitere sind ebenso denkbar, beispielsweise, dass Strahlabschwächungen in abgestuften Zwischenstufen erzeugt werden.

Beispielsweise zeigt die Figur 3a ein Linienmuster, das horizontal oder vertikal angeordnet ist und harte Übergänge zwischen Feldern aufweist. Ein derartiges Modulatorfeld weist einen rechteckigen Grauwert-Verlauf auf, wie er beispielsweise in Figur 3a auf der rechten Seite dargestellt ist.

Figur 3b zeigt ein Linienmuster, das horizontal oder vertikal angeordnet sein kann, wobei ein derartiges Linienmuster in Wellenform angeordnet ist, d. h. Übergänge zwischen Bereichen unterschiedlicher Röntgenstrahlungsabschwächungskoeffizienten sind "weich". Auf der rechten Seite der Figur 3b ist der dazugehörige Sinus-modulierte Grauwert-Verlauf dargestellt.

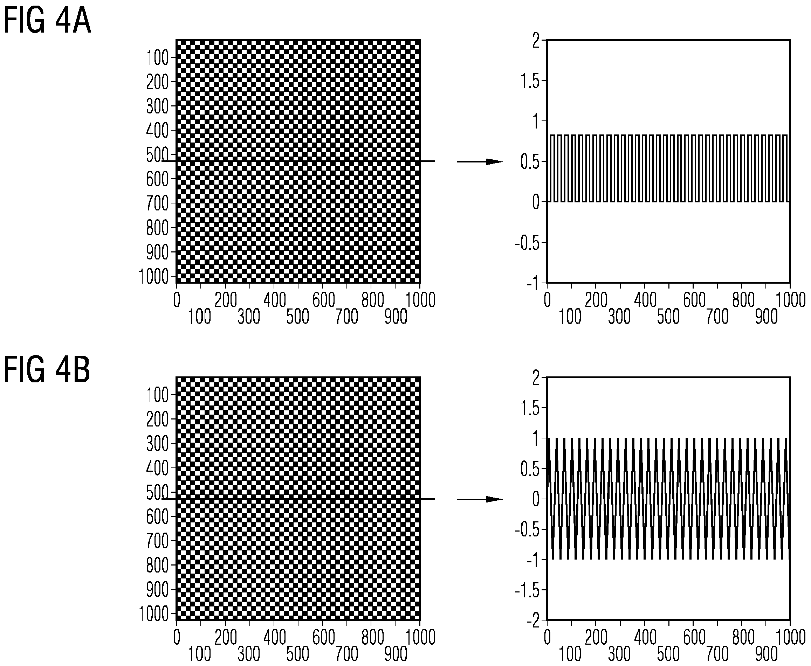

Figur 4a zeigt ein schachbrettartiges Modulatorfeld, mit "harten" Übergängen, d. h. der auf der rechten Seite der Figur 4a dargestellte Grauwert-Verlauf ist rechteckig.

Figur 4b zeigt ein weiteres schachbrettartiges Modulatorfeld mit "weichen" Übergängen, d. h. ein in dieser Figur 4b rechts dargestellter Grauwert-Verlauf ist Sinus-moduliert.

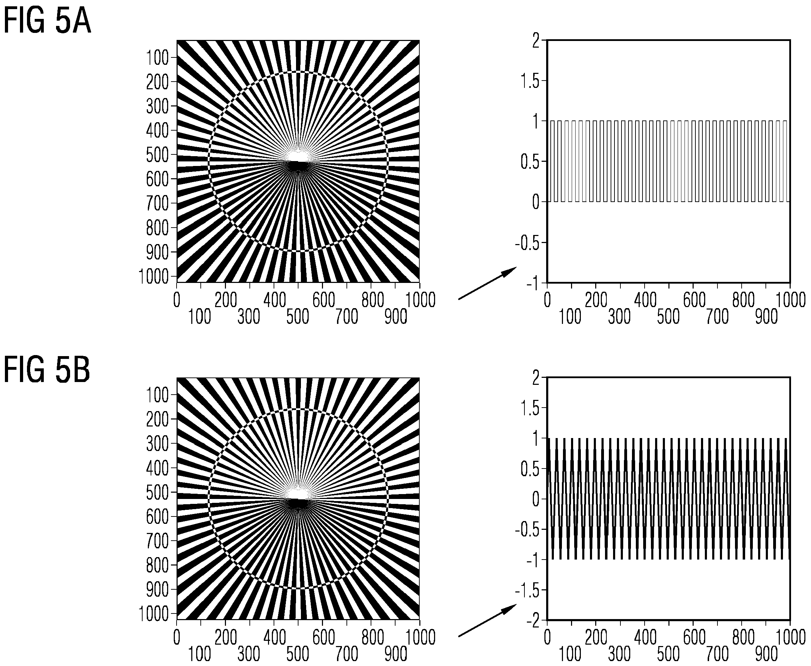

Figur 5a zeigt ein Radialmuster, das beispielsweise als "Chopper-Rad" bezeichnet werden kann, mit "harten" Übergängen, die sich aus einem in Figur 5a rechts dargestellten rechteckigen Grauwert-Verlauf ergeben.

Figur 5b zeigt ein weiteres Ausführungsbeispiel eines Modulatorfeldes mit einem Radialmuster, bei dem ein Sinus-modulierter Grauwert-Verlauf ausgebildet ist, so wie er in Figur 5b rechts dargestellt ist. Bei Verwendung eines Radialmusters wird eine Verschiebeeinrichtung als Rotationsmotor zur Drehung des Modulatorfeldes um eine Rotationsachse bereitgestellt. Verschiedene Aspekte für einen Aufbau eines Primärmodulators oder Modulatorfeldes sind zu berücksichtigen. Eine Modulationsstärke gibt an, mit welcher Stärke ein Primärsignal maximal abgeschwächt wird. Im Falle eines Schachbrettmodulators entspricht dies der Dicke der entsprechenden dunklen Kupferfelder. Die Modulationsstärke hat Einfluss einerseits auf die Genauigkeit, mit der die Streuwerte abgeschätzt werden, andererseits ist durch diese auch festgelegt, wie viel Primärinformation an diesen Stellen verlorengeht. Da eine Verbesserung der Messgenauigkeit der Streuwerte eine Abnahme an Primärinformation, d. h. eine Zunahme des Rauschens, bedeutet, ist hier ein Kompromiss zu wählen. Derzeit liegen bevorzugte Modulationsstärken bei einer Gesamtsignal-Verringerung im Bereich von ca. 10 bis 30% bei den dunklen Feldern. Weiterhin wird berücksichtigt, dass der Primärmodulator so aufgebaut sein sollte, dass über die genannten j Modulationsprojektionen hinweg jedes Pixel der gleichen aufaddierten Gesamtintensität ausgesetzt ist, um auf die Fläche gesehen ein gleichmäßiges Verhalten zu erzielen. Dies ist keine notwendige Voraussetzung für eine korrekte Messung, erleichtert aber die Auswertung der Gesamtstrahlung. In diesem Fall kann direkt mit der aufaddierten Intensität gerechnet werden, andernfalls ist eine weitere Korrektur notwendig, die man durch eine Messung ohne Objekt unter exakt gleichen Bedingungen erhält. Technisch am leichtesten zu realisieren ist die Bedingung der Gleichmäßigkeit, wenn eine Verschiebung des Modulatorfeldes in den Modulationsprojektionen exakt eine Periode des Modulators abdeckt. Des Weiteren ist es für ein erfindungsgemäßes Verfahren wesentlich, dass sich eine Streuverteilung und eine Streuverteilungssituation während einer zeitlichen Modulation eines Primärsignals möglichst nicht ändern. Denn eine Änderung der Streusituation innerhalb der j Modulationsprojektionen führt zu einer zunehmend fehlerhaften Abschätzung der Streuwerte. Hinsichtlich erfindungsgemäßer Primärmodulatoren ist es besonders vorteilhaft, wenn sichergestellt ist, dass sich die Streuverteilung und die Streuverteilungssituation während einer zeitlichen Primärmodulation praktisch gesehen möglichst lediglich geringfügig ändern wird. Dazu sollte die Primärmodulation möglichst kleinflächig und in benachbarten Bereichen phasenversetzt erfolgen. Im Idealfall würde die zeitliche Primärmodulation für jeden Pixel einzeln und dabei mit einer Phasenverschiebung um Pi zwischen benachbarten Pixeln erfolgen. Zusätzlich sollte die Modulationsstärke möglichst gering ausfallen, obwohl dies konträr zu dem Bestreben ist, eine möglichst große Modulationsstärke zu realisieren, um die Streuwerte möglichst genau zu messen, so wie dies vorstehend beschrieben wurde. Derartige Anforderungen werden beispielsweise mittels eines relativ feinen Schachbrettmusters mit einer einem gewünschten Rauschen angepasster Modulationsstärke annähernd erreicht. Eine Modulationsstärke kann dabei anhand des Rauschens optimiert werden. Zwischen den Extremsituationen unbrauchbares rauschendes Streubild und unbrauchbares, weil zu stark verändertes Streubild, kann eine optimale Stärke ausgewählt werden, bei der die Artefakte durch Streuung minimiert werden. Dieses kann beispielsweise mittels Simulation oder Testmessungen mit unterschiedlich starken Modulatoren bewirkt werden.

Grundsätzlich ist vom Schutzbereich dieser Anmeldung jede technische Maßnahme zur zeitlichen Modulation eines Primär-signals umfasst. Eine weitere Ausführungsform einer zeitlichen Modulation kann derart bereitgestellt sein, dass bereits innerhalb einer Röntgenquelle einem Röntgenstrahl ein steuerbares Muster aufgeprägt wird, das zeitlich steuerbar und/oder veränderbar ist. Es sollte sichergestellt sein, dass eine Modulation möglichst kleinflächig erfolgt, wobei eine reine Modulation eines Gesamtröhrenstroms jedoch nicht ausreichend ist, da eine Kleinflächigkeit nicht gegeben ist.

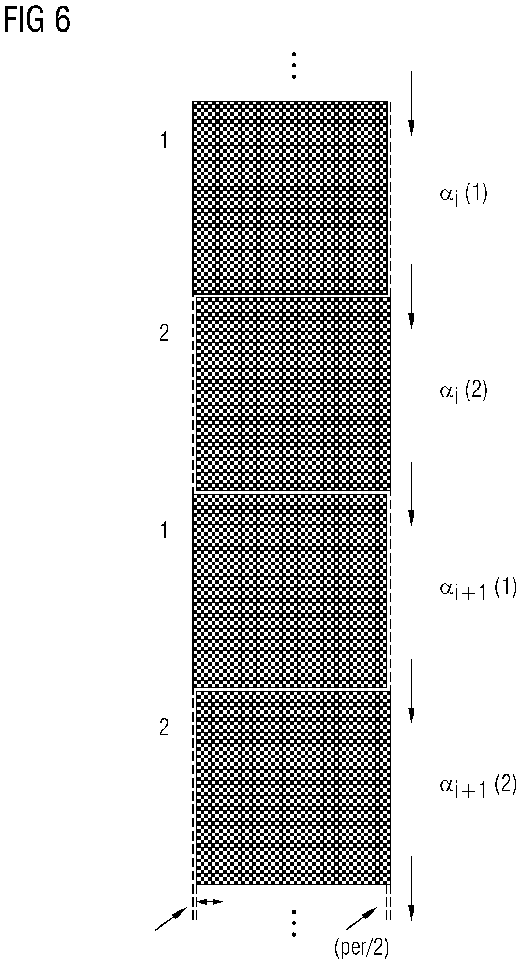

Figur 6 zeigt ein Ausführungsbeispiel einer Modulatorfeldbewegung und dazu relativen Objektbewegung; es wird eine Bewegung bzw. ein Versatz, der beispielsweise horizontal oder vertikal oder diagonal sein kann, des primär modulierenden Modulatorfeldes um eine halbe Periodenlänge per/2 bereitgestellt, und zwar derart, dass für jede Winkelstellung des zu tomographierenden Objektes zwei, um per/2 versetzte Projektionen vorhanden sind. Dazu wird das Modulatorfeld nicht stationär installiert, wie dies gemäß der US 7,463,712 B2 offenbart ist, sondern wird auf einem motorisierten Lineartisch um mindestens eine halbe Periodenlänge beweglich vorgesehen. Innerhalb einer CT-Abtastung wird das Modulatorfeld dann für jede Winkelstellung $\alpha_i$ (i = 1 ... n, wobei n die Anzahl der aufzunehmenden Winkelstellungen bedeutet) des zu tomographierenden Objekts nach einer ersten Projektionsaufnahme in einer ersten Modulatorposition 1 (Projektion $\alpha_i(1)$) um eine halbe Periodenlänge derart verfahren, dass im Falle eines schachbrettartigen Modulatorfeldes die dunklen Felder dort zu liegen kommen, wo vorher helle Felder waren und umgekehrt. Es wird dann eine zweite Projektion (bei der zweiten Modulatorposition 2 (Projektion $\alpha_i(2)$) aufgezeichnet, wobei das zu tomographierende Objekt noch nicht bewegt wurde. Für die nächste Winkelstellung $\alpha_{i+1}$ des zu tomographierenden Objekts und alle folgenden wird diese zweifache Aufnahmeprozedur mit der ersten Modulatorfeldposition 1 und der zweiten Modulatorfeldposition 2 ebenso wiederholt. Die vertikalen Pfeile entsprechen einem Zeitstrahl. Dieser Satz an zwei Modulationsprojektionen stellt pixelweise/für jeden einzelnen Pixel die zeitliche Modulation des Primärsignals, überlagert mit dem möglichst unmodulierten Streusignal, dar. Diese Gesamtsignale werden dann jeweils hochpassgefiltert, wodurch der niederfrequente Streuanteil entfernt wird. Anschließend erfolgt eine Demodulation durch Multiplikation des hochpassgefilterten Gesamtsignals mit der entsprechenden Modulationsfunktion, die als Ergebnis ein abgeschätztes Primärsignal liefert. Um zu einem Streusignal zu gelangen, wird dieses abgeschätzte Primärsignal von dem addierten, gewichteten Gesamtsignal abgezogen. Das berechnete Streubild wird nachfolgend von der ursprünglichen, modulierten CT-Projektion, die insbesondere nicht unterabgetastet ist, subtrahiert. Es folgt dann eine pixelweise Strahlaufhärtungskorrektur, die nachfolgend näher beschrieben wird. Das streustrahl- und strahlaufhärtungskorrigierte Bild kann zur Bereinigung des Modulatormusters schließlich durch eine reine Modulatorprojektion, d.h. ohne Objekt im Strahlengang dividiert werden. Die vorstehend beschriebenen Schritte werden für beide Projektionen $\alpha_i(1)$ und $\alpha_i(2)$ in der ersten Modulatorposition 1 und der zweiten Modulatorposition 2 ausgeführt. Da das Objekt in diesen beiden Projektionen nicht bewegt wurde, werden zur Verbesserung des Signal-Rausch-Verhältnisses (SNR) die streustrahl- und strahlaufhärtungs- und modulatorkorrigierten Projektionen gemittelt.

Figur 7 zeigt ein Ausführungsbeispiel einer Korrektur von Strahlaufhärtungseffekten, die ebenso als Beam Hardening (BH) bezeichnet werden. Es erfolgt eine einfache Strahlaufhärtungskorrektur für dunkle Felder, wobei eine derartige Korrektur in ähnlicher Weise bereits für eine herkömmliche CT angewendet wird, dort aber für komplette CT-Projektionen. In dem erfindungsgemäßen Fall der Primärmodulation tritt ein verstärkter Strahlaufhärtungs-Effekt an den Stellen der dunklen Felder auf, da hier das zusätzliche Material, das beispielsweise Kupfer oder Wolfram sein kann, eine zusätzliche Aufhärtung des Strahls bewirkt, die bei den hellen Feldern nicht auftritt. Durch eine einfache Simulation ist es möglich, theoretisch exakte Abschwächungskurven für bestimmte Aufnahmeparameter zu simulieren (siehe dazu [1]). Eine bekannte Einschränkung dieser Methode liegt darin, dass bei der im Folgenden vorgestellten Korrektur die theoretische Exaktheit nur für Monomaterialien zutrifft, d.h. lediglich für aufzunehmende Objekte bestehend aus einem Material, das beispielsweise Aluminium sein kann. Werden Objekte, bestehend aus einer Vielzahl an Materialien, die als Multimaterialien bezeichnet werden, untersucht, besteht eine mehr oder weniger große Diskrepanz zu den tatsächlich auftretenden Aufhärtungseffekten. In Figur 7 sind zwei Abschwächungskurven $I/I_0$ für das Beispiel folgender Aufnahmeparameter dargestellt: Röhrenspannung 200 kV, röhrenseitiger Vorverstärker von 2 mm Kupfer und Objektmaterial Aluminium. Dabei zeigen die beiden Kurven das Verhältnis des aufgenommenen Signals zum Gesamtsignal, wobei jeweils eine Normierung auf 1 ausgeführt wurde. Das Verhältnis des aufgenommenen Signals zum Gesamtsignal ist gegen die ansteigende Durchstrahlungslänge durch einen Aluminiumkeil aufgetragen. Dabei zeigt im linken Graph die untere Kurve den Fall der hellen Felder, bei dem die Vorfilterung des Spektrums aus tatsächlich nur 2,0 mm Kupfer besteht. Die obere Kurve hingegen gibt den Fall der dunklen Felder wieder, wobei ein Gesamtfilter, bestehend aus 2,7 mm Kupfer angenommen wird. D.h. zusätzlich zu dem röhrenseitigen Vorfilter von 2,0 mm Stärke wird das zusätzliche Material des Modulators in den dunklen Feldern, in diesem Fall 0,7 mm Kupfer, berücksichtigt. Die Eingangsintensitäten, also die Intensitäten, die nach dem Modulator, aber vor dem Objekt vorliegen, sind für die dunklen Felder kleiner als für die hellen Felder. Dennoch sind die Spektren der Strahlung hinter den dunklen Feldern im Mittel energiereicher, also stärker aufgehärtet als die entsprechenden hinter den hellen Feldern. Aufgrund dieses Unterschiedes zwischen den Spektren, durchstrahlt die Strahlung hinter den dunklen Feldern wirksamer das nachfolgende Objektmaterial, und zwar relativ, also auf die gleiche Eingangsintensität bezogen. Dieser Einfluss wird nachfolgend berücksichtigt, indem für die dunklen Felder dieser Effekt mittels Rechnung kompensiert wird. Es ergibt sich für das stärker gefilterte 2,7 mm-Cu-Spektrum (obere Kurve) eine relativ gesehen kleinere Abschwächung als mit schwächer gefiltertem 2,0 mm-Cu-Spektrum (untere Kurve). Gemäß einer ersten Ausführungsform wird eine Strahlaufhärtungskorrektur vor der Streuabschätzung ausgeführt. Die dunklen Felder des modulierten Projektionsbildes werden beim Unterabtastschritt strahlaufhärtungskorrigiert, d.h. der in einem dunklen Feld (m, n) gemessene Wert UnkorrWert (m, n) wird durch einen Wert korr-Wert, der wie folgt berechnet wird:

$$(1) \; \texttt{korrWert (m, n) = BHC[UnkorrWert(m, n)-ApproxStreuWert}$$
$$\texttt{(m, n)]+ApproxStreuWert(m, n)}$$

Von dem gemessenen Wert UnkorrWert (m, n) wird zunächst ein approximierter Streuwert ApproxStreuWert (m, n) abgezogen, der aus dem zuletzt berechneten Streubild der vorangegangenen Projektion erzeugt wird. Dieser Wert wird in der Abschwächungskurve für die dunklen Felder (obere Kurve) gesucht, er korrespondiert mit einer gewissen Durchstrahlungslänge. Bei dieser Durchstrahlungslänge lässt sich nun ein entsprechender Abschwächungswert für die hellen Felder (untere Kurve) finden. Dieser Schritt wird in der Gleichung als Funktion BHC[.] dargestellt. Zu diesem strahlaufhärtungskorrigierten Wert wird zuletzt noch das approximierte Streusignal ApproxStreuWert (m, n) wieder hinzuaddiert. Damit ist, theoretisch, der Strahlaufhärtungs-Effekt für das behandelte dunkle Feld (m, n) vollständig kompensiert.

In Figur 7 ist zusätzlich zu den Abschwächungskurven in einem Koordinatensystem mit durchstrahlter Aluminiumlänge beliebiger Einheit entlang der Abszisse und $I/I_0$ als normierte Projektionsintensität entlang der Ordinate ein zweiter Graph dargestellt, der für die dunklen Felder die Korrekturwerte, d.h. die Differenz der beiden vorstehend beschriebenen Abschwächungskurven, in Grauwerten gegenüber einem ursprünglichen Grauwert in einem dunklen Feld angibt. Entsprechend kann ebenso aus dem zweiten Graph ausgehend von einem ursprünglichen Grauwert in einem dunklen Feld, dieser durch Subtraktion des Korrekturwertes korrigiert werden.

Gemäß einer weiteren vorteilhaften Ausführungsform der vorliegenden Erfindung kann die Korrektur von Strahlaufhärtungseffekten ebenso nach der Streuabschätzung, und vor der Division durch das reine Modulatorbild ausgeführt werden. Dazu müssen für eine vollständig korrigierte CT-Projektion nach der Streuabschätzung noch folgende Schritte ausgeführt werden. Von der ursprünglichen modulierten CT-Projektion wird das soeben berechnete Streubild subtrahiert. Für die dunklen Felder wird dann pixelweise eine Strahlaufhärtungskorrektur ausgeführt, wie dies in Verbindung mit Formel (1) beschrieben ist. Dabei wird für jedes Pixel einzeln berücksichtigt, wie viel zusätzlich aufhärtendes Material durch den Modulator bzw. das Modulatorfeld vorhanden ist. Gemäß dem Ausführungsbeispiel sind dies zwischen 0,0 und 0,7 mm zusätzliches Kupfer. Auf diese Weise können bei Verwendung eines Kegelstrahl-Computertomographen ebenso die Randpixel der dunklen Felder optimal korrigiert werden, denn bei einer derartigen Anlagengeometrie sind die Ränder der dunklen Felder insbesondere für dezentral liegende Felder nicht scharf begrenzt, sondern weisen vielmehr einen allmählichen Grauwertverlauf auf.

Der Einsatz einer Strahlaufhärtungs-Korrektur an zwei Stellen im erfindungsgemäßen Verfahren verbessert wesentlich erstens eine Streuabschätzung, und zwar insbesondere bei Monomaterialien, bei der ersten Alternative einer Strahlaufhärtungs-Korrektur und zweitens führt er zu einer vollständigeren Korrektur eines Modulatorfeldmusters bei einem Divisionsschritt durch die Modulatorfeldprojektion bzw. Modulationsprojektion, und zwar bei der zweiten Alternative einer Strahlaufhärtungs-Korrektur, wodurch wiederum Ringartefakte im CT-Volumen wirksam verringert werden können.

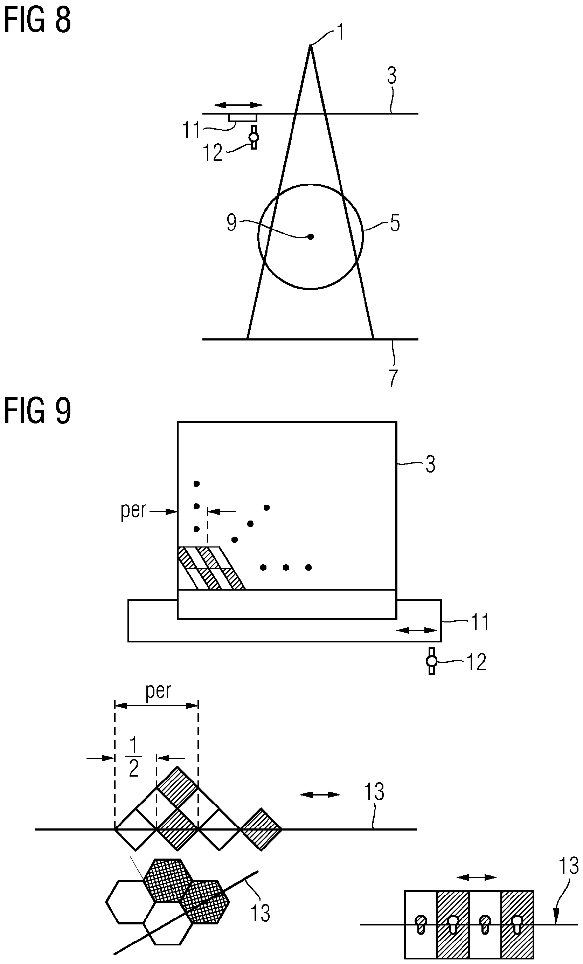

Figur 8 zeigt ein Ausführungsbeispiel einer erfindungsgemäßen Vorrichtung. Ein Primärsignal beziehungsweise eine Röntgenstrahlung, die als Dreieck dargestellt ist, einer primären Röntgenquelle 1 durchläuft ein Modulatorfeld 3 mit einem sich wiederholenden Muster von Bereichen mit unterschiedlicher Röntgenstrahlungsabschwächung. Anschließend durchläuft die Strahlung ein abzubildendes Objekt 5, das um eine Rotationsachse 9 drehbar positioniert ist. Das Ergebnis ist eine ursprüngliche amplitudenmodulierte Projektion auf einem Detektor 7.

Figur 9 zeigt ein Ausführungsbeispiel eines erfindungsgemäßen Modulatorfeldes. Grundsätzlich kann eine Verschiebeeinrichtung zur Bereitstellung einer Relativbewegung zwischen Röntgenquelle, Objekt und Detektor einerseits und Modulatorfeld andererseits ein Linearmotor für Modulatorfelder vergleichbar mit denen gemäß Figuren 3a bis 4b oder ein Rotationsmotor für Modulatorfelder vergleichbar mit denen gemäß Figuren 5a und 5b sein. Im Falle eines linearen Modulatorfeldes bewegt ein Linearmotor 12 einen Lineartisch 11, der das Modulatorfeld 3 positioniert. Ein erfindungsgemäßes Modulatorfeld 3 weist ein sich wiederholendes Muster von Bereichen mit zwei unterschiedlichen Röntgenstrahlungs-Abschwächungskoeffizienten auf. Eine erste Hälfte des sich wiederholenden Musters ist deckungsgleich mit einer zweiten Hälfte, wobei zueinander deckungsgleiche Bereiche der beiden Hälften zueinander entgegen gesetzte Röntgenstrahlungs-Abschwächungskoeffizienten aufweisen, sich das Muster entlang mindestens einer Wiederholungslinie 13 wiederholt und eine Länge des Musters entlang der Wiederholungslinie 3 einer Periodenlänge per entspricht. Figur 9 zeigt, dass bei einer Verschiebebewegung des Modulatorfeldes 3 von einer ersten Position in eine zweite Position entlang der Wiederholungslinie 13 um beispielsweise eine halbe Periodenlänge, Modulatorfeldbereiche mit kleinen und dazu relativ großen Röntgenstrahl-Abschwächungskoeffizienten gegenseitig ausgewechselt werden. Gemäß der vorliegenden Anmeldung sind grundsätzlich alle Muster möglich, die das vorstehend beschriebene Auswechseln bereitstellen. Es eignen sich insbesondere Muster, die Vielecke aufweisen. Figur 9 zeigt Ausführungsbeispiele von Modulatorfeldmustern, wie beispielsweise Parallelogramme, Rauten und Sechsecke. Des Weiteren sind bei Verwendung von Vielecken Hälften des Musters verwendbar, die jeweils beliebige Formen innerhalb der Vielecke aufweisen.

## Patentansprüche

1.  Verfahren zur Korrektur von Artefakten in einer Röntgenprojektion eines Objektes, wobei eine Röntgenstrahlung einer primären Röntgenquelle mit einem sich wiederholenden Muster von Bereichen mit unterschiedlicher Röntgenstrahlungssignalstärke beaufschlagt, dadurch amplitudenmoduliert wird, danach durch das abzubildende Objekt zu einem Detektor verläuft, dort als Gesamtsignale erfasst und daraus ein Streubild berechnet wird, das von einer ursprünglichen amplitudenmodulierten Projektion getrennt wird, wobei
    die Röntgenstrahlung der primären Röntgenquelle Primärsignale erzeugt, **dadurch gekennzeichnet dass** und diese zeitlich veränderlich amplitudenmoduliert werden, so dass eine Anzahl ($N_{Mod}$) von mindestens zwei von einem jeweils unterschiedlichen Zeitpunkt zugeordneten Modulationsprojektionen erzeugt werden, und dass zur Berechnung eines einer Projektion zugeordneten Streubildes für jeden Detektorpixel ein Streusignal des Detektorpixels mittels Subtraktion eines nach der zeitlichen Modulation demodulierten Primärsignals des Detektorpixels von dem Gesamtsignal des Detektorpixels erhalten wird und alle auf diese Weise ermittelten Streuwerte für alle Detektorpixel zusammengesetzt das Streubild ergeben.

2.  Verfahren nach Anspruch 1,
    **dadurch gekennzeichnet, dass** die primäre Röntgenquelle einzeln ansteuerbare Matrixbereiche aufweist, die zu einem vorangehenden Zeitpunkt entsprechend dem Muster eine kleine oder eine dazu relativ große und zu einem nachfolgenden Zeitpunkt die jeweils andere Röntgenstrahlungssignalstärke aussenden und zu jedem der Zeitpunkte jeweils eine ursprüngliche amplitudenmodulierte Modulationsprojektion des Objekts erzeugt und jeweils ein dieser Modulationsprojektion zugeordnetes Streubild ermittelt wird.

3.  Verfahren nach Anspruch 1,
    **dadurch gekennzeichnet, dass** im Strahlenverlauf von der Röntgenquelle zum Objekt ein dem Muster entsprechende Bereiche mit einer kleinen oder einer dazu relativ großen Röntgenstrahlungsabschwächung aufweisendes Modulatorfeld angeordnet ist, und Röntgenquelle, Objekt und Detektor einerseits und das Modulatorfeld andererseits von einer vorangehenden Position in eine nachfolgende Position relativ zueinander bewegt werden und dadurch Modulatorfeldbereiche mit kleinem und dazu relativ großem Röntgenstrahlungsabschwächungskoeffizienten im Röntgenstrahlungsverlauf gegenseitig ausgewechselt werden, in jeder der beiden Positionen jeweils eine ursprüngliche amplitudenmodulierte Modulationsprojektion des Objekts erzeugt und ein diesen Modulationsprojektionen zugeordnetes Streubild ermittelt wird.

4.  Verfahren nach Anspruch 1, 2 oder 3,
    **dadurch gekennzeichnet, dass** die Demodulation mittels Multiplikation des aufgenommenen Gesamtsignals mit einer Modulationsfunktion ausgeführt wird.

5.  Verfahren nach Anspruch 1, 2, 3 oder 4,

**gekennzeichnet durch** eine zur Bereitstellung einer streukorrigierten Modulationsprojektion erfolgende Subtraktion des der Modulationsprojektion zugeordneten Streubildes von einer dazugehörigen ursprünglichen, nicht unterabgetasteten Modulationsprojektion.

6. Verfahren nach Anspruch 5,
   **gekennzeichnet durch** eine zur Bereitstellung einer zusätzlich modulationskorrigierten Modulationsprojektion erfolgende Division der streukorrigierten Modulationsprojektion durch eine ohne das Objekt ermittelte zeitliche Modulationsfunktion.

7. Verfahren nach einem der vorangehenden Ansprüche 5 oder 6, **gekennzeichnet durch** Erzeugen jeweils einer streukorrigierten oder streu- und modulatorfeldkorrigierten Modulationsprojektion jeweils für eine Anzahl $j = 1 \ldots N_{Mod}$ Modulationen mit darauf folgender Mittelung zur Vergrößerung eines Signal-Rausch-Verhältnisses.

8. Verfahren nach einem der vorangehenden Ansprüche,
   **dadurch gekennzeichnet, dass** die vorangehende und nachfolgende Modulation jeweils eine Modulationsprojektion j=1 und j=2 erzeugen und danach Röntgenquelle und Detektor einerseits und das Objekt andererseits nach jeder zweiten Modulationsprojektion um eine Drehachse in eine Drehrichtung um einen Drehwinkelschritt von einer Relativposition i in eine Relativposition i+1 relativ zueinander gedreht werden.

9. Verfahren nach Anspruch 8,
   **dadurch gekennzeichnet, dass** zur Berechnung eines einer Modulationsprojektion zugeordneten Streubildes ein Unterabtastungsschritt ausgeführt wird.

10. Verfahren nach Anspruch 8 oder 9,
    **dadurch gekennzeichnet, dass** zur Berechnung eines einer Modulationsprojektion zugeordneten und für ein jedes Pixel ein einzelnes Primärsignal aufweisenden Primärbildes, für jedes Pixel das Gesamtsignal über alle Modulationsprojektionen j=1...N_ModProj hinweg als zeitliches Signal gewertet und dieses Hochpass gefiltert und danach mittels Multiplikation mit der jeweiligen dem Pixel eigenen Modulationsfunktion demoduliert und danach Tiefpass gefiltert wird.

11. Verfahren nach Anspruch 10,
    **dadurch gekennzeichnet, dass** zur Berechnung eines einer Modulationsprojektion zugeordneten Streubildes das der Modulationsprojektion zugeordnete demodulierte Primärbild von einem gemittelten Gesamtbild subtrahiert wird.

12. Verfahren nach einem der vorangehenden Ansprüche,
    **dadurch gekennzeichnet, dass** zur Strahlaufhärtungskorrektur jeweils für ein Modulationsprojektionsbildfeld, das durch eine relativ kleine Röntgenstrahlungssignalstärke erzeugt wurde, von einem gemessenen unkorrigierten Intensitätswert ein angenäherter Streuwert subtrahiert wird, diese Differenz einem ersten Intensitätswert entspricht, der mittels einer dazugehörigen Intensitätsabschwächungskurve einer dazugehörigen durchstrahlten Objektlänge zugeordnet wird, diese mittels einer Intensitätsabschwächungskurve für ein Modulationsprojektionsbildfeld, das durch eine relativ große Röntgenstrahlungssignalstärke erzeugt wurde, einem zweiten Intensitätswert zugeordnet wird, zu dem der angenäherte Streuwert wieder addiert wird.

13. Verfahren nach Anspruch 12,
    **dadurch gekennzeichnet, dass** der angenäherte Streuwert aus einem berechneten Streubild einer vorangegangenen ursprünglichen Modulationsprojektion ermittelt wurde.

14. Verfahren nach Anspruch 12 oder 13,
    **dadurch gekennzeichnet, dass** die Strahlaufhärtungskorrektur während des Unterabtastschrittes nach Anspruch 10 ausgeführt wird.

15. Verfahren nach Anspruch 12 oder 13,
    **dadurch gekennzeichnet, dass** die Strahlaufhärtungskorrektur vor einer optionalen Division der streukorrigierten Modulationsprojektion durch die ohne das Objekt ermittelte zeitliche Modulationsfunktion nach Anspruch 6 ausgeführt wird.

16. Computertomograph zur Korrektur von Artefakten in einer Röntgenprojektion eines Objektes, wobei eine Röntgenstrahlung einer primären Röntgenquelle des Computertomographen durch ein Modulatorfeld des Computertomographen mit einem sich wiederholenden Muster von Bereichen mit unterschiedlicher Röntgenstrahlungsabschwächung verläuft, dadurch amplitudenmoduliert wird, danach durch das abzubildende Objekt zu einem Detektor des Computertomographen verläuft, dort erfasst und daraus ein Streubild berechnet wird, das von einer ursprünglichen amplitudenmodulierten Projektion getrennt wird, wobei das Modulatorfeld ein sich wiederholendes Muster aufweist, dessen erste Hälfte deckungsgleich mit einer zweiten Hälfte ist, zueinander deckungsgleiche Bereiche der beiden Hälften zueinander entgegen gesetzte Röntgenstrahlungsabschwächungskoeffizienten aufweisen, sich das Muster entlang mindestens einer Wiederholungslinie wiederholt und eine Länge des Musters entlang der Wiederholungslinie einer Periodenlänge entspricht; **gekennzeichnet durch**

eine Verschiebeeinrichtung zur Relativbewegung der Röntgenquelle, des Objekts und des Detektors einerseits und des Modulatorfeldes andererseits relativ zueinander von einer vorangehenden Position in eine nachfolgende Position entlang der Wiederholungslinie derart, dass Modulatorfeldbereiche mit kleinem und dazu relativ großem Röntgenstrahlungsabschwächungskoeffizienten im Röntgenstrahlungsverlauf mindestens einmal gegenseitig ausgewechselt werden,

wobei der Computertomograph dazu ausgebildet ist, ein Verfahren nach einem der Ansprüche 1 bis 15 durchzuführen.

17. Computertomograph nach Anspruch 16,
**dadurch gekennzeichnet, dass** die Verschiebeeinrichtung und eine Dreheinrichtung zum Drehen des Objekts um eine Drehachse in eine Drehrichtung um jeweilige Drehwinkelschritte miteinander synchronisiert sind.

18. Computertomograph nach einem der Ansprüche 16 oder 17,
**dadurch gekennzeichnet, dass** das Muster ein Linien-, Vier eck-, Wellen- oder Radialmuster ist.

19. Computertomograph nach einem der Ansprüche 16 bis 18,
**dadurch gekennzeichnet, dass** sich Röntgenstrahlungsabschwächungskoeffizienten von Übergängen der Modulatorfeldbereiche mit kleinem zu den Modulatorfeldbereichen mit relativ großem Röntgenstrahlungsabschwächungskoeffizienten kontinuierlich und stetig verändern.

20. Computertomograph nach einem der Ansprüche 16 bis 19,
**dadurch gekennzeichnet, dass** Modulationsstärken der Modulatorfeldbereiche mit relativ großem Röntgenstrahlungsabschwächungskoeffizienten eine Gesamtsignal-Verringerung von 10% bis 30% bewirken.

21. Computertomograph nach einem der Ansprüche 16 bis 20,
**dadurch gekennzeichnet, dass** das Modulatorfeld Bereiche mit kleinem und dazu relativ großem sowie mit zusätzlichen Röntgenstrahlungsabschwächungskoeffizienten aufweist.

## Claims

1. Method for correcting artefacts in an X-ray projection of an object, wherein X-rays of a primary X-ray source are charged with a repeating pattern of regions with different X-ray signal strengths, is thus amplitude modulated, then run through the object to be imaged to a detector, is here detected as a complete signal, and from this a scattered image is calculated, which is separated from an original, amplitude-modulated projection,
wherein the X-rays of the primary X-ray source generate primary signals, **characterised in that** these are amplitude modulated with temporal alterations, such that a number ($N_{Mod}$) of at least two modulation projections allocated to a respective different point in time is generated and that to calculate a scattered image allocated to a projection for each detector pixel, a scattering signal of the detector pixel is obtained by subtracting a primary signal of the detector pixel that has been demodulated after the temporal modulation from the complete signal of the detector pixel and all scattered values determined in this way produce the scattered image for all detector pixels combined.

2. Method according to claim 1,
**characterised in that** the primary X-ray source has individually activateable matrix regions which, at a previous point in time according to the pattern, emit a small or a relatively large X-ray signal strength, and at a subsequent point in time emit the respective other X-ray signal strength, and at every point in time a respective original amplitude-modulated modulation projection of the object is generated and a respective scattered image allocated to this modulation projection is determined.

3. Method according to claim 1,

**characterised in that** a modulator field having regions corresponding to the pattern with a small or a relatively large X-ray attenuation thereto is arranged in the beam path from the X-ray source to the object, and X-ray source, object and detector on the one hand, and the modulator field on the other, are moved from a previous position to a subsequent position relative to each other and thus modulator field regions with small and relatively large X-ray attenuation coefficients thereto are mutually exchanged in the X-ray beam path, a respective original amplitude-modulated modulation projection of the object is generated in each of the two positions and a scattered image allocated to these modulation projections is determined.

4. Method according to claim 1, 2 or 3,
**characterised in that** the demodulation is embodied by means of multiplication of the recorded complete signal with a modulation function.

5. Method according to claim 1, 2, 3 or 4,
**characterised by** a subtraction of the scattered image assigned to the modulation projection, which takes place to provide a scatter-corrected modulation projection, being carried out by a corresponding original modulation projection that has not been underscanned.

6. Method according to claim 5,
**characterised by** a division of the scatter-corrected modulation projection carried out for the provision of an additionally modulation-corrected modulation projection being effected by a temporal modulation function that is determined without the object.

7. Method according to one of the preceding claims 5 or 6,
**characterised by** generation of a respective scatter-corrected or scatter and modulator-field-corrected modulation projection for a respective number $j = 1 \ldots N_{Mod}$ of modulations with subsequent averaging for increasing a signal-to-noise ratio.

8. Method according to one of the preceding claims
**characterised in that** the previous and subsequent modulation can generate a respective modulation projection j=1 and j=2 and then the X-ray source and detector on the one hand and the object on the other hand can be rotated relative to each other around an axis of rotation in a rotational direction at an angle of rotation step from a relative position i to a relative position i+1.

9. Method according to claim 8,
**characterised in that** an underscanning step is carried out for the calculation of a scattered image allocated to a modulation projection.

10. Method according to claim 8 or 9,
**characterised in that**, for calculating a primary image allocated to a modulation projection and having an individual primary signal for each pixel, the complete signal is evaluated for each pixel over all modulation projections j=1...N_ModProj as a temporal signal, and this is high-pass filtered and then demodulated by means of multiplication with the respective modulation function corresponding to the pixel, and then is low-pass filtered.

11. Method according to claim 10,
**characterised in that** the demodulated primary image allocated to the modulation projection is subtracted from an averaged complete image to calculate a scattered image assigned to a modulation projection.

12. Method according to one of the preceding claims
**characterised in that** an approximated scattering value is subtracted from a measured, uncorrected intensity value for beam-hardening correction for a respective modulation projection image field, which has been generated by a relatively small X-ray signal strength; this difference corresponds to a first intensity value, which is allocated to a corresponding penetrated object length by means of a corresponding intensity attenuation curve; this is allocated to a second intensity value by means of an intensity attenuation curve for a modulation projection image field, which has been generated by a relatively high X-ray signal strength, to which intensity value the approximated scattering value is re-added.

13. Method according to claim 12,
**characterised in that** the approximated scattering value has been determined from a calculated scattered image of a previous original modulation projection.

**14.** Method according to claim 12 or 13,
**characterised in that** the beam-hardening correction is carried out during the underscanning step according to claim 10.

**15.** Method according to claim 12 or 13,
**characterised in that** the beam-hardening correction is embodied by the temporal modulation function according to claim 6, which is determined without the object, before an optional division of the scatter-corrected modulation projection.

**16.** Computed tomography device for correcting artefacts in an X-ray projection of an object, wherein X-rays of a primary X-ray source of the computed tomography device run through a modulator field of the computed tomography device with a repeating pattern of regions with different X-ray attenuation, are amplitude modulated here, then run through the object to be imaged to a detector of the computed tomography device, detected there and a scattered image is calculated from this, which is separated from an original, amplitude-modulated projection, wherein the modulator field has a repeating pattern, the first half of which is congruent to a second half, regions of both halves, which are congruent to each other, have X-ray attenuation coefficients that are opposite to each other, the pattern is repeated along at least one repeating line and a length of the pattern along the repeating line corresponds to a period length;
**characterized by** a displacement apparatus for the relative movement of the X-ray source, the object and the detector on the one hand and the modulator field on the other hand, relative to one another from a previous position to a subsequent position along the repeating line, in such a way that modulator field regions with small and relatively large X-ray attenuation coefficients thereto are mutually exchanged in the X-ray path at least once,
wherein the computed tomography device is embodied to carry out a method according to one of claims 1 to 15.

**17.** Computed tomography device according to claim 16, **characterised in that** the displacement apparatus and a rotational apparatus are synchronized with each other in order to rotate the object around an axis of rotation in a rotational direction at respective angle of rotation steps.

**18.** Computed tomography device according to one of claims 16 or 17,
**characterised in that** the pattern is a linear, square, wavelike or radial pattern.

**19.** Computed tomography device according to one of claims 16 to 18,
**characterised in that** X-ray attenuation coefficients are changed continuously and consistently from transitions of the modulator field regions with small X-ray attenuation coefficients to those with relatively large X-ray attenuation coefficients.

**20.** Computed tomography device according to one of claims 16 to 19,
**characterised in that** modulation strengths of the modulator field regions with a relatively large X-ray attenuation coefficient effect a complete signal reduction of 10% to 30%.

**21.** Computed tomography device according to one of claims 16 to 20,
**characterised in that** the modulator field has regions with a small and relatively large X-ray attenuation coefficient thereto, as well as with additional X-ray attenuation coefficients.

**Revendications**

**1.** Procédé de correction d'artéfacts dans une radioprojection d'un objet, dans lequel un rayonnement X d'une source primaire de rayons X est alimenté en un modèle récurrent de régions d'intensité du signal du rayonnement X différente, en étant ainsi modulé en amplitude, puis va, en passant à travers l'objet à reproduire, à un détecteur, y est détecté comme signaux d'ensemble et on en calcule une image de dispersion, qui est séparée d'une projection d'origine modulée en amplitude, dans lequel le rayonnement X de la source primaire de rayons X produit des signaux primaires, **caractérisé en ce que** ceux-ci sont modulés en amplitude de manière variable dans le temps, de manière à produire un nombre ($N_{Mod}$) d'au moins deux projections de modulation associées à, respectivement, un instant différent et
**en ce que**, pour calculer une image de dispersion associée à une projection, on obtient, pour chaque pixel du détecteur, un signal de dispersion du pixel du détecteur, en soustrayant un signal primaire démodulé, après la modulation dans le temps, du pixel du détecteur du signal d'ensemble du pixel du détecteur et toutes les valeurs de dispersion ainsi déterminées, rassemblées pour tous les pixels du détecteur, donnent l'image de dispersion.

2. Procédé suivant la revendication 1,
**caractérisé en ce que** la source primaire de rayons X a des régions de matrice individuelles pouvant être commandées, qui, à un instant précédent, conformément au modèle, envoient une intensité de signal de rayonnement X petite ou, par rapport à cela, relativement grande, et à un instant suivant, l'autre intensité de signal de rayons X et, à chacun des instants, on produit, respectivement, une projection de modulation d'origine modulée en amplitude de l'objet et on détermine, respectivement, une image de dispersion associée à cette projection de modulation.

3. Procédé suivant la revendication 1,
**caractérisé en ce que**, dans le trajet de rayonnement de la source de rayons X à l'objet, est disposé un champ de modulateur ayant des régions, conformément au modèle, d'affaiblissement petit, ou par rapport à cela, relativement grand du rayonnement X et on déplace la source de rayons X, l'objet et le détecteur d'une part et le champ de modulateur d'autre part, d'une position précédente à une position suivante, les uns par rapport aux autres, et on échange ainsi mutuellement des régions du champ de modulateur, ayant un petit coefficient d'affaiblissement du rayonnement X et, par rapport à cela, relativement grand, dans le trajet du rayonnement X, dans chacune des deux positions on produit respectivement une projection de modulation d'origine modulée en amplitude de l'objet et on détermine une image de dispersion associée à ces projections de modulation.

4. Procédé suivant la revendication 1, 2 ou 3,
**caractérisé en ce que** l'on réalise la démodulation au moyen d'une multiplication du signal d'ensemble enregistré par une fonction de modulation.

5. Procédé suivant la revendication 1, 2, 3 ou 4,
**caractérisé par** une soustraction, s'effectuant pour mettre à disposition une projection de modulation corrigée en dispersion, de l'image de dispersion, associée à la projection de modulation, d'une projection de modulation d'origine en relation et non sous-échantillonée.

6. Procédé suivant la revendication 5,
**caractérisé par** une division, s'effectuant pour mettre à disposition une projection de modulation corrigée supplémentairement en modulation, de la projection de modulation corrigée en dispersion par une fonction de modulation dans le temps déterminée sans l'objet.

7. Procédé suivant l'une des revendications précédentes 5 ou 6,
**caractérisé par** la production respectivement d'une projection de modulation, corrigée en dispersion et en champ de modulateur, respectivement pour un nombre $j = 1...N_{Mod}$ de modulations avec formation ensuite d'une moyenne pour augmenter un rapport signal-bruit.

8. Procédé suivant l'une des revendications précédentes,
**caractérisé en ce que** la modulation précédente et suivante produisent chacune une projection de modulation j=1 et j=2 et, ensuite, la source de rayons X et le détecteur d'une part et l'objet d'autre part sont, après une projection de modulation sur deux, tournés autour d'un axe de rotation dans un sens de rotation d'un pas d'angle de rotation, en passant d'une position i relative à une position i+1 relative l'un par rapport à l'autre.

9. Procédé suivant la revendication 8,
**caractérisé en ce que**, pour calculer une image de dispersion associée à une projection de modulation, on effectue un stade de sous-échantillonnage.

10. Procédé suivant la revendication 8 ou 9,
**caractérisé en ce que**, pour calculer une image primaire, associée à une projection de modulation et ayant un signal primaire individuel pour chaque pixel, on évalue, pour chaque pixel, le signal d'ensemble sur toutes les projections de modulation j=1...N_Mod Proj de modulation, comme signal en fonction du temps et on le filtre passe haut et, ensuite, on le démodule au moyen d'une multiplication par la fonction de modulation respective propre au pixel et, ensuite, on le filtre passe bas.

11. Procédé suivant la revendication 10,
**caractérisé en ce que**, pour calculer une image de dispersion associée à une projection de modulation, on soustrait l'image primaire démodulée, associée à la projection de modulation, d'une image d'ensemble, dont on a fait la moyenne.

12. Procédé suivant l'une des revendications précédentes,
**caractérisé en ce que**, pour corriger la dureté du rayonnement respectivement pour un champ d'image de projection de modulation, qui a été produit par une intensité de signal de rayonnement X relativement petite, on soustrait, d'une valeur d'intensité mesurée non corrigée, une valeur de dispersion approximative, cette différence correspondant à une première valeur d'intensité, qui est associée au moyen d'une courbe d'affaiblissement d'intensité associée à une longueur d'objet traversée associée, celle-ci est, au moyen d'une courbe d'affaiblissement d'intensité pour un champ d'image de projection de modulation, qui a été produit par une intensité de signal de rayonnement X relativement grande, associée à une deuxième valeur d'intensité à laquelle la valeur de dispersion approximative est ajoutée à nouveau.

13. Procédé suivant la revendication 12,
**caractérisé en ce que** la valeur de dispersion approximative a été déterminée à partir d'une image de dispersion calculée d'une projection de modulation d'origine précédente.

14. Procédé suivant la revendication 12 ou 13,
**caractérisé en ce que** l'on effectue la correction de dureté du rayonnement pendant le stade de sous-échantillonnage suivant la revendication 10.

15. Procédé suivant la revendication 12 ou 13,
**caractérisé en ce que** l'on effectue la correction de dureté du rayonnement avant une division éventuelle de la projection de modulation corrigée en dispersion, par la fonction de modulation en fonction du temps déterminé sans l'objet suivant la revendication 6.

16. Tomographe à ordinateur pour corriger des artéfacts dans une radioprojection d'un objet, un rayonnement X d'une source primaire de rayons X du tomographe à ordinateur s'étendant en ayant un motif récurrent de régions d'affaiblissement du rayonnement X différent, en étant ainsi modulée en amplitude, allant ensuite, en passant à travers l'objet à reproduire, à un détecteur du tomographe à ordinateur, y étant détecté et une image de dispersion en étant calculée, qui est séparée d'une projection modulée en amplitude d'origine, le champ de modulateur ayant un motif récurrent, dont la première moitié est en coïncidence avec une deuxième moitié, des parties en coïncidence entre elles des deux moitiés ayant des coefficients d'affaiblissement du rayonnement X opposés l'un à l'autre, le motif se répétant le long d'au moins une ligne de récurrence et une longueur du motif correspondant à une période le long de la ligne de récurrence, **caractérisé par** un dispositif de déplacement pour le déplacement relatif de la source de rayons X, de l'objet et du détecteur d'une part et du champ de modulateur d'autre part, les uns par rapport aux autres, d'une position précédente à une position suivante, suivant la ligne de récurrence, de manière à ce que des régions du champ de modulateur, ayant des coefficients d'affaiblissement du rayonnement X petits et, par rapport à cela, relativement grands, soient échangées dans le trajet du rayonnement X mutuellement au moins une fois, dans lequel le tomographe à ordinateur est constitué pour effectuer un procédé suivant l'une des revendications 1 à 15.

17. Tomographe à ordinateur suivant la revendication 16,
**caractérisé en ce que** le dispositif de déplacement et un dispositif de mise en rotation, pour faire tourner l'objet autour d'un axe de rotation, dans un sens de rotation de pas d'angle de rotation respectifs, sont synchronisés entre eux.

18. Tomographe à ordinateur suivant l'une des revendications 16 ou 17,
**caractérisé en ce que** le motif est un motif linéaire, quadrangulaire, ondulé ou radial.

19. Tomographe à ordinateur suivant l'une des revendications 16 à 18,
**caractérisé en ce que** des coefficients d'affaiblissement du rayonnement X se modifient continuellement et constamment de transition des régions du champ de modulateur, ayant un coefficient d'affaiblissement du rayonnement X petit, aux régions du champ de modulateur, ayant des coefficients d'affaiblissement du rayonnement X relativement grands.

20. Tomographe à ordinateur suivant l'une des revendications 16 à 19,
**caractérisé en ce que** des intensités de modulation des régions du champ de modulateur, ayant des coefficients d'affaiblissement du rayonnement X relativement grands, provoquent une diminution du signal d'ensemble de 10% à 30ù.

21. Tomographe à ordinateur suivant l'une des revendications 16 à 20,
**caractérisé en ce que** le champ de modulation a des régions ayant des coefficients d'affaiblissement du rayonnement X petits et, par rapport à cela, relativement grands, ainsi que des coefficients d'affaiblissement du rayonnement X supplémentaires.

## FIG 1A

i-1       i       i+1       i+2

## FIG 1B

$(j=1...N_{ModProj.})$

$j=1$       $j=2$       $j=3$       $j=N_{ModProj.}$

FIG 1C

FIG 1D

FIG 1E

EP 2 645 934 B1

# FIG 2

E

$-f_{Mod}$    0    $f_{Mod}$    f

A

B

C

A          _          C  =  D

FIG 3A

FIG 3B

FIG 4A

FIG 4B

FIG 5A

FIG 5B

## FIG 6

FIG 7

EP 2 645 934 B1

FIG 8

FIG 9

per

per

$\frac{1}{2}$

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 7463712 B2
- DE 2454537

**In der Beschreibung aufgeführte Nicht-Patentliteratur**

- **KOWALSKI, G.** Suppression of scattered radiation in radiography and improvement of resolution by spatially modulated intensity. *Applied Optics USA,* Marz 1976, vol. 15 (3), ISSN 0003-6935, 648-655